# FASCICULE DE BREVET EUROPEEN

(11) **EP 0 970 961 B1**
(45) Date de publication et mention de la délivrance du brevet: **25.04.2001**
(21) Numéro de dépôt: 99401354.8
(22) Date de dépôt: 04.06.1999
(51) Int. Cl.: C07F 7/08, C07F 7/21, A61K 7/42

(54) **Procédé de photostabilisation de filtres solaires dérivés du dibenzoylméthane, compositions cosmétiques filtrantes photostabilisées ainsi obtenues et leurs utilisations**
Photostabilisierung von Dibenzoylderivat-Sonnenschutzverbindungen, diese enthaltende Sonnenschutzzusammensetzungen, und deren Verwendung
Photostabilisation of dibenzoylmethane-based sunscreens, cosmetic sunscreen compositions thus obtained, and their use

(30) Priorité: 10.07.1998 FR 9808937
(43) Date de publication de la demande: 12.01.2000
(73) Titulaire: L'OREAL, 75008 Paris (FR)
(72) Inventeur: Richard, Hervé, 93240 Villepinte (FR); Forestier, Serge, 77410 Claye Souilly (FR)
(74) Mandataire: Miszputen, Laurent

(56) Documents cités:
- EP-A- 0 335 777
- EP-A- 0 712 855
- WO-A-93/10745
- WO-A-94/04131

## Description

La présente invention se rapporte à un nouveau procédé pour améliorer la stabilité d'au moins un dérivé du dibenzoylméthane vis-à-vis du rayonnement UV consistant à associer au dit dérivé de dibenzoylméthane une quantité efficace d'un composé silanique ou organosiloxanique à fonction benzylidène camphre

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'absorption intrinsèque. Ces dérivés du dibenzoylméthane, qui sont maintenant des produits bien connus en soi à titre de filtres actifs dans l'UV-A, sont notamment décrits dans les demandes de brevets français FR-A-2326405 et FR-A-2440933, ainsi que dans la demande de brevet européen EP-A-0114607 ; le 4-(ter.-butyl) 4'-méthoxy dibenzoylméthane est par ailleurs actuellement proposé à la vente sous la dénomination commerciale de "®PARSOL 1789" par la Société GIVAUDAN.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que, de façon contraignante, des applications répétées à intervalles de temps réguliers et rapprochés doivent être effectuées par l'utilisateur pour obtenir une protection efficace de la peau contre les rayons UV.

Ainsi, il a été proposé dans le document EP-A-0709080 d'associer aux dérivés du dibenzoylméthane des dérivés de benzalmalonate afin de diminuer la photoinstabilité desdits dérivés de dibenzoylméthane. Toutefois, la photostabilisation des dérivés du dibenzoylméthane vis-à-vis du rayonnement UV constitue, à ce jour, un problème qui n'a pas encore été résolu de manière complètement satisfaisante.

Il a été également proposé dans les documents FR-A-2607996 et WO 94/04131 d'associer aux dérivés du dibenzoylméthane des dérivés hydrocarbonés de benzylidène camphre tel que le 3-(4-methylbenzylidène)-camphre afin de diminuer la photoinstabilité desdits dérivés de dibenzoylméthane.

Une autre difficulté, indépendante de celle évoquée ci-avant, rencontrée avec les dérivés de dibenzoylméthane est qu'il s'agit de filtres lipophiles présentant la particularité mais aussi le désavantage d'être solides à température ambiante. De ce fait, leur utilisation dans une composition cosmétique antisolaire implique certaines contraintes au niveau de leur formulation et de leur mise en oeuvre, en particulier lorsqu'il s'agit de trouver des solvants permettant de les solubiliser correctement, seuls ou conjointement avec d'autres filtres. A cet égard, on fait aujourd'hui le plus souvent appel à des huiles comme des esters et plus particulièrement des benzoates d'alkyles en C₁₂-C₁₅ ("®FINSOLV TN" de chez Finetex), ou à des triglycérides et notamment des triglycérides d'acides gras en C₈-C₁₂ ("®MIGLYOL 812" de chez Hüls), mais ces différents produits possèdent des propriétés solubilisantes vis à vis des filtres susmentionnés qui peuvent apparaître comme encore insuffisantes.

Les formulations antisolaires à base de dérivés de dibenzoylméthane et de 3-(4-methylbenzylidène)-camphre telles que mises en oeuvre dans les documents FR-A-2607996 et WO 94/04131 ne permettent pas de manière satisfaisante de résoudre ce problème de solubilité desdits dérivés de dibenzoylméthane.

On connaît dans l'art antérieur un certain nombre de composés silaniques ou organosiloxaniques à fonction benzylidènecamphre dans les demandes de brevet EP-A-712 855, EP-A-0335 777 et WO93/10745. Or, la Demanderesse vient maintenant de découvrir, de façon surprenante, qu'en associant aux dérivés du dibenzoylméthane mentionnés ci-dessus une quantité efficace d'un composé silanique ou organosiloxanique à fonction benzylidène camphre, il était possible d'améliorer de manière substantielle et remarquable, la stabilité photochimique (ou photostabilité) de ces mêmes dérivés du dibenzoylméthane.

Il a été également trouvé, et il s'agit là de l'un des avantages supplémentaires attachés à la présente invention, que les composés silaniques ou organosiloxaniques à fonction benzylidène camphre utilisables dans le cadre de la présente invention à titre d'agents photostabilisants constituent par ailleurs, de façon également très surprenante par rapport aux filtres organiques hydrocarbonés dérivés de benzylidène camphre connus, des solvants particulièrement remarquables pour les filtres du type dérivés du dibenzoylméthane comme par exemple le 4-(ter.-butyl) 4'-méthoxy dibenzoylméthane ; ce qui permet, à quantité égale de solvant, de mettre en oeuvre des quantités plus importantes de filtres. D'autre part, il a été également trouvé, que la photostabilité des dérivés du dibenzoylméthane obtenue en présence des filtres organiques hydrocarbonés du type benzylidène camphre connus pouvait être encore améliorée avec les composés silaniques ou organosiloxaniques à fonction benzylidène camphre utilisables selon la présente invention.

Ainsi, conformément à un premier objet de la présente invention, il est proposé un nouveau procédé pour améliorer la stabilité d'au moins un dérivé du dibenzoylméthane vis-à-vis du rayonnement UV, consistant à associer au dit dérivé du dibenzoylméthane une quantité efficace d'un dérivé silanique ou organosiloxanique à fonction benzylidène camphre.

Les compositions cosmétiques et/ou dermatologiques obtenues conformément au procédé de l'invention présentent l'avantage d'être particulièrement photostables, même après une exposition prolongée aux rayonnements UV-A et UV-B. Ces rayonnements peuvent être d'origine naturelle (soleil) ou artificielle (lampe UV).

La présente invention a également pour objet l'utilisation d'un composé silanique ou organosiloxanique à fonction benzylidène camphre dans la préparation d'une composition cosmétique ou dermatologique contenant au moins un dérivé du dibenzoylméthane, pour améliorer la stabilité dudit dérivé du dibenzoylméthane vis-à-vis du rayonnement UV.

D'autres caractéristiques, aspects et avantages de la présente invention apparaîtront à la lecture de la description détaillée qui va suivre.

Comme indiqué précédemment, les dérivés du dibenzoylméthane destinés à être photostabilisés selon la présente invention sont des produits déjà bien connus en soi et décrits notamment dans les documents FR-A-2326405, FR-A-2440933. et EP-A-0114607 précités.

Selon la présente invention, on peut bien entendu mettre en oeuvre un ou plusieurs dérivés du dibenzoylméthane.

Parmi les dérivés du dibenzoylméthane utilisables selon la présente invention, on peut notamment citer, de manière non limitative :
- le 2-méthyldibenzoylméthane,
- le 4-méthyldibenzoylméthane,
- le 4-isopropyldibenzoylméthane,
- le 4-tert,-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "®PARSOL 1789" par la Société GIVAUDAN, ce filtre répondant donc à la formule développée suivante : 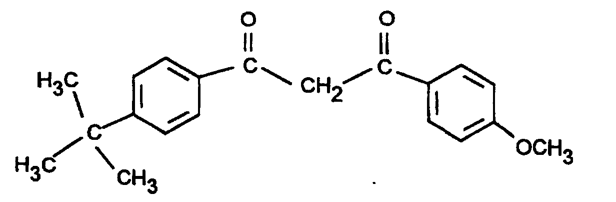 Un autre dérivé du dibenzoylméthane préféré selon la présente invention est le 4-isopropyl-dibenzoylméthane, filtre vendu sous la dénomination de "®EUSOLEX 8020" par la Société MERCK, et répondant à la formule développée suivante : 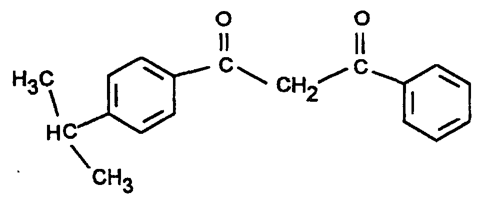 Le ou les dérivés du dibenzoylméthane peuvent être présents dans les compositions destinées à être stabilisées conformément au procédé de l'invention, à des teneurs allant généralement de 0,01 % à 10 % en poids, et de préférence à des teneurs allant de 0,3 % à 5 % en poids, par rapport au poids total de la composition.

Un deuxième composé des compositions selon l'invention est un composé silanique ou organosiloxanique à fonction benzylidène camphre. Ces demiers sont bien connus et sont par exemple décrits et synthétisés dans les documents EP-A-0325881, EP-A-0335777 et EP-A-0712855 (ces documents font partie intégrante du contenu de la demande).

De préférence, le composé silanique ou organosiloxane à groupement benzylidène camphre utilisé dans la présente invention répond à la formule (I) suivante : 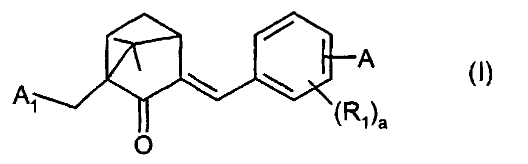 dans laquelle :
- R₁, identiques ou différents, représentent l'hydrogène, un radical OH, un radical alkyle saturé ou non, linéaire ou ramifié, en C₁-C₁₀, un radical alcoxy linéaire ou ramifié en C₁-C₁₀ ou -OSi(CH₃)₃, deux R₁ adjacents pouvant former ensemble un groupement alkylidène dioxy dans lequel le groupe alkylidène contient de 1 à 2 atomes de carbone ;
- a est un nombre entier allant de 1 à 3 ;
- A est l'hydrogène ou un radical de formule -L-W tel que :
- L est un radical divalent de formules (IIa) ou (IIb) suivantes :

   -CH₂-CHR₂-[C(R₃)₂]ₘ-(CO)ₙ-(O)ₒ-(Z)ₚ-(Y)_{q}- (IIa)

   -CH=CR₂-[C(R₃)₂]ₘ-(CO)ₙ-(O)ₒ-(Z)ₚ-(Y)_{q}- (IIb)

   dans laquelle :
   - R₂ et R₃, identiques ou différents, représentent l'hydrogène ou un radical alkyle linéaire ou ramifié en C₁-C₅,
   - Z est un radical di-yle en C₁-C₆, linéaire ou ramifié, saturé ou insaturé, éventuellement substitué par un radical hydroxyle ou alcoyle en C₂-C₈, linéaire ou ramifié, saturé ou insaturé,
   - Y représente -O-, -NR₄-, -SO₂NH-, -(CO)O-, -(CO)NH- ou -O(CO)NH-, avec R₄ qui représente l'hydrogène ou un radical alkyle en C₁-C₅ ;
   - m est un nombre entier allant de 0 à 10 ;
   - n est 0 ou 1 ;
   - o est 0 ou 1 ;
   - p est 0 ou 1 ;
   - q est 0 ou 1 ;
   - A₁ est l'hydrogène ou le radical -L₁-W dans lequel le radical L₁ a la définition de L sous réserve que lorsque q = 1 alors Y représente -SO₂NH- et étant entendu qu'un seul des deux radicaux A et A₁ est l'hydrogène ;
   - W est un radical de formules (1), (2) ou (3) suivantes : 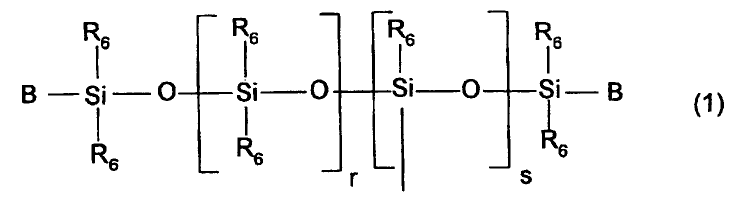 ou

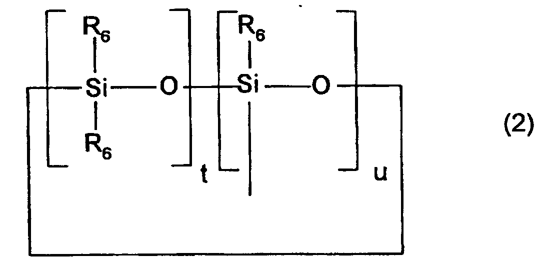

      -Si(R₆)₃ (3)

      dans lesquelles :
      - R₆, identiques ou différents, sont choisis parmi les radicaux alkyles linéaires ou ramifiés en C₁-C₃₀, phényle, au moins 80% en nombre des radicaux R₆ étant méthyle ;
      - B, identiques ou différents sont choisis parmi les radicaux R₆ et le radical X de formule suivante : 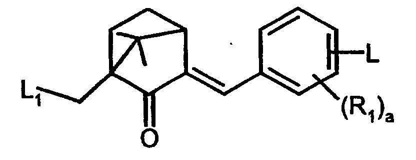 dans laquelle R₁, L, L₁ et a ont les mêmes significations que ci-dessus ;
      - r est un nombre entier compris entre 0 et 200 inclusivement, et s est un nombre entier compris entre 0 et 50 inclusivement, et si s = 0, au moins l'un des deux symboles B désigne X ;
      - u est un nombre entier compris entre 1 et 10 inclusivement, et t est un nombre entier compris entre 0 et 10 inclusivement, étant entendu que t + u est égal ou supérieur à 3.

Parmi les composés ci-dessus on peut plus particulièrement citer la famille des composés présentant au moins l'une, de préférence l'ensemble, des caractéristiques suivantes :
- R₁ = H, OCH₃, CH₃ ou deux radicaux R₁ adjacents forment un méthylène dioxy,
- R₂ = H ou CH₃,
- n = 0,
- q = 1.

Au sein de cette famille, on utilise de préférence les composés présentant au moins l'une, de préférence l'ensemble, des caractéristiques suivantes :
- R₆ est méthyle,
- B est méthyle,
- r est compris entre 5 et 20 inclus,
- s est compris entre 2 et 15 inclus,
- t + u est compris entre 3 et 10 inclus,
- m = 1,
- R₃ est l'hydrogène ou un radical méthyle.

Ces composés ainsi que leur procédé de préparation sont par exemple décrits dans le document EP-A-0335777.

Une deuxième famille de composés préférés est celle regroupant les composés de formule (I) où s = 0.

A titre d'exemples de composés de formule (I) particulièrement préférés, on citera les produits de formules suivantes :

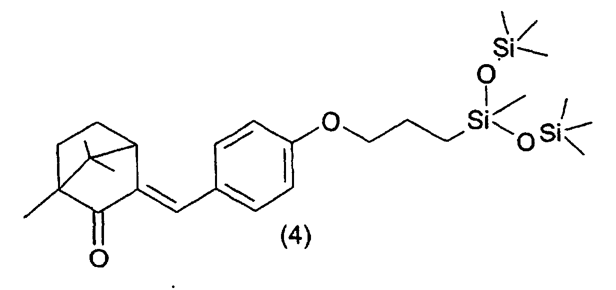

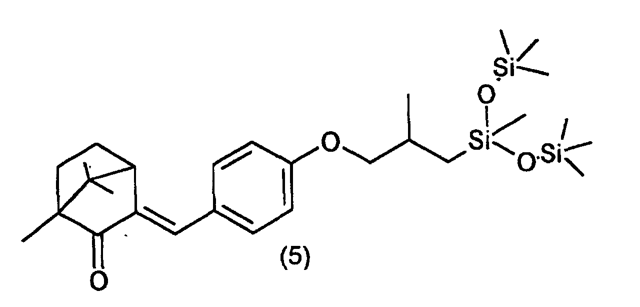

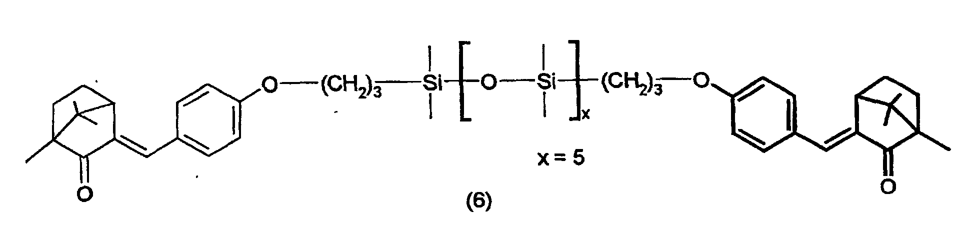

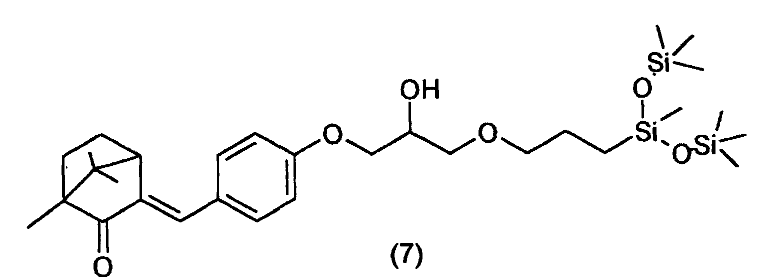

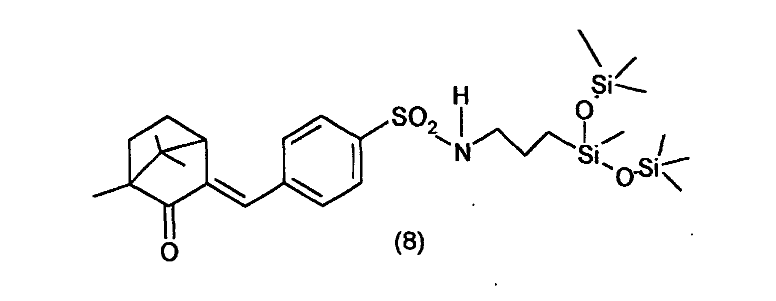

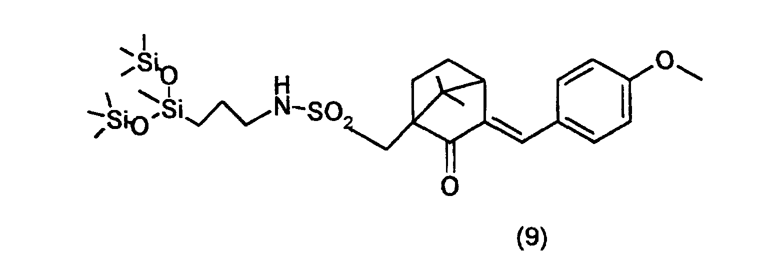

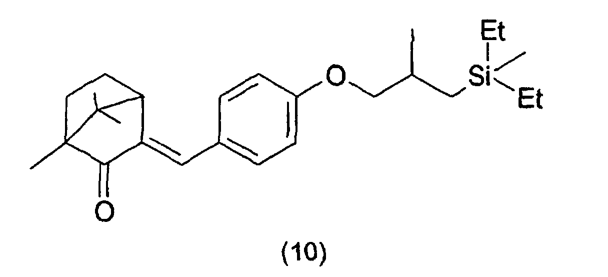

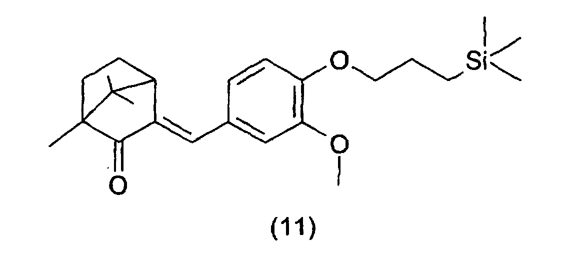

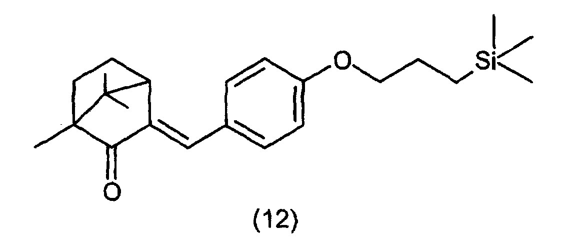

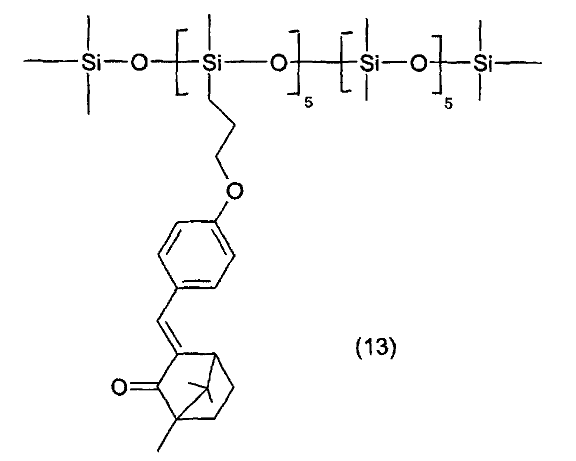

Parmi les composés de formule (I) tels que définis ci-dessus, certains d'entre eux sont nouveaux et constituent un autre objet de la présente invention.

C'est le cas des composés de formule (I) telle que définie ci-dessus dans laquelle p est 1 et le radical Z est un radical di-yle en C₁-C₆ linéaire ou ramifié, saturé ou insaturé et possédant un radical hydroxyle. Parmi ces composés particuliers, on peut citer le composé (7) tel que décrit précédemment.

Une autre famille de composés de formule (I) nouveaux est constituée par les composés (10), (11) et (12) tels que décrits précédemment.

Par quantité efficace de composé silanique ou organosiloxane à fonction benzylidène camphre selon l'invention, on entend une quantité suffisante pour obtenir une amélioration notable et significative de la photostabilité du ou des dérivés du dibenzoylméthane contenus dans la composition. La quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que décrit dans la demande FR-A-2607700.

Les composés silaniques ou organosiloxanes à fonction benzylidène camphre sont généralement présents dans les compositions selon l'invention à une teneur au moins égale à 0,5 % en poids, par rapport au poids total de la composition. De préférence encore, cette teneur va de 0,5 % à 20 % en poids, par rapport au poids total de la composition.

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles. Ces filtres complémentaires peuvent être notamment choisis parmi les dérivés cinnamiques, les dérivés salicyliques, les dérivés de benzophénones, les dérivés de benzotriazole, les dérivés de benzimidazole, les dérivés de triazine, les dérivés du benzalmalonate, les dérivés de β,β'-diphénylacrylate, les dérivés de l'acide p-aminobenzoïque, les polymères filtres et silicones filtres décrits dans la demande WO-9304665. D'autres exemples de filtres organiques sont donnés dans la demande de brevet EP-A-0487404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A-0518773.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti-radicaux libres, les opacifiants, les stabilisants, les émollients, les silicones, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions. Bien entendu, tous les ingrédients supplémentaires susceptibles d'être introduits dans les compositions conformes à l'invention doivent être tels qu'ils ne perturbent ou n'altèrent substantiellement pas l'effet de photostabilisation exercé par les composés silaniques ou organosiloxaniques à fonction benzylidène camphre sur les dérivés du dibenzoylméthane.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « ®Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de camauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR-A-2315991 et FR-A-2416008).

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, et peut constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Des exemples concrets illustrant l'invention, vont maintenant être donnés.

### EXEMPLE 1 :

### Préparation du dérivé de formule (4) :

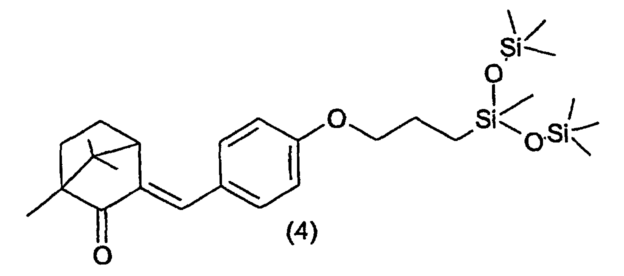

A une solution de 3-(4-allyloxy-benzylidène)-1,7,7-trimethyl-bicyclo[2.2.1]heptan-2-one préparé conformément à l'exemple 8 de FR-A-2430938 (50 g, 0,169 mole) et de catalyseur (complexe à 3-3.5% poids de Pt dans du cyclovinylméthylsiloxane de Hüls ®Petrarch PC085 : 200 µl) dans 140 ml de toluène sec porté à 80°C, on ajoute goutte à goutte en 30 minutes 37,53 g (0,169 mole) d'heptaméthyltrisiloxane. On laisse à cette température pendant 6 heures. On concentre le mélange réactionnel. On reprend dans le dichlorométhane et passe cette solution sur un lit de ®Célite. L'huile jaune pâle obtenue est cristallisée dans l'heptane. On obtient ainsi 31 g (Rendement : 41 %) du dérivé de l'exemple 1 sous forme d'une poudre blanche:
Pf : 47-48°C
-UV (Ethanol à 95%) λₘₐₓ = 318 nm, εₘₐₓ = 27 000

| -Analyse élémentaire pour C₂₇ H₄₆ O₄ Si₃ | | | |
|---|---|---|---|
| théorie | C62.50 | H8.94 | Si16.24 |
| trouvé | C62.43 | H9.00 | Si16.26 |

### EXEMPLE 2 :

### Préparation du dérivé de formule (5) :

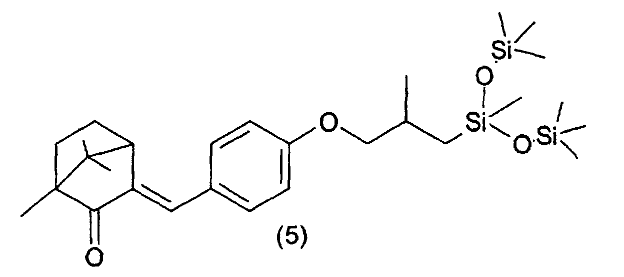

A une solution de 1,7,7-Trimethyl-3-[4-(2-methyl-allyloxy)-benzylidène]-bicyclo[2.2.1] heptan-2-one (15,5 g, 0,05 mole) et de catalyseur (complexe à 3-3.5% poids de Pt dans du cyclovinylméthylsiloxane de Hüls Petrarch PC085 : 100 µl) dans 35 ml de toluène sec porté à 80°C, on ajoute goutte à goutte en 30 minutes 12,2 g (0,055 mole) d'heptaméthyltrisiloxane. On laisse à cette température pendant 3 heures. On concentre le mélange réactionnel et on obtient après chromatographie sur silice (éluant : heptane/ dichlorométhane 50:50) 23 g (Rendement : 86 %) d'une huile incolore du dérivé de l'exemple 2 :
-UV (Ethanol) λₘₐₓ = 320 nm, εₘₐₓ = 26 200

| - Analyse élémentaire pour C₂₈ H₄₆ O₄ Si₃ | | | |
|---|---|---|---|
| théorie | C63.10 | H9.08 | Si15.81 |
| trouvé | C63.02 | H9.00 | Si15.64 |

### EXEMPLE 3 :

### Préparation du dérivé de formule (6) :

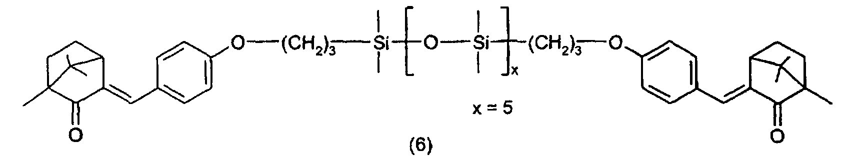

A une solution de 1,7,7-Trimethyl-3-[4-(2-methyl-allyloxy)-benzylidène]-bicyclo[2.2.1] heptan-2-one (69,8 g, 0,235 mole) et de catalyseur (complexe à 3-3.5% poids de Pt dans du cyclovinylméthylsiloxane de Hüls Petrarch PC085 : 100 µl) dans 110 ml de toluène sec porté à 80°C, on ajoute goutte à goutte en 1 heure 30 minutes 50 g (0,117 mole) de α,ω-dodécaméthyl hexasiloxane. On laisse à cette température pendant 1 heure 30 minutes. On concentre le mélange réactionnel et on obtient après chromatographie sur silice (éluant : heptane/acétate d'éthyle 98:2 puis gradient jusque heptane/acétate d'éthyle 50:50) 47,5 g (Rendement : 40 %) d'une huile visqueuse jaune pâle du dérivé de l'exemple 3 :
-UV (Ethanol) λₘₐₓ = 320 nm, εₘₐₓ = 26 200

| - Analyse élémentaire pour C₅₂ H₈₆ O₉ Si₆ | | | |
|---|---|---|---|
| théorie | C61.01 | H8.47 | Si16.46 |
| trouvé | C61.04 | H8.52 | Si16.10 |

### EXEMPLE 4 :

### Préparation du dérivé de formule (7) :

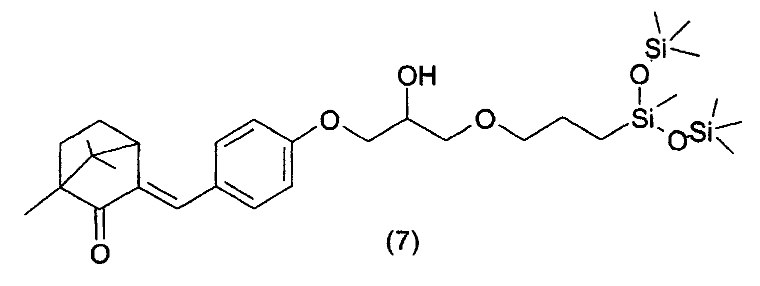

On porte à 110 °C sous barbotage d'azote pendant 6 heures, un mélange de 3-(4-hydroxy-benzylidène)-1,7,7-trimethyl-N -bicyclo[2.2.1]heptan-2-one (10 g, 0,039 mole) et de 3-glycidyloxypropyl-Bis-(triméthylsiloxy)-méthyl silane (14,8 g, 0,044 mole) en présence de bromure de tetrabutyl ammonium (0,6 g). L'huile brute obtenue a été chromatographiée sur Silice (éluant : acétate d'éthyle/heptane 20:80) pour donner en fractions milieux 10 g (Rendement : 43 %) du dérivé de l'exemple 4 sous forme d'une huile incolore :
-UV (Ethanol) λₘₐₓ = 318 nm, εₘₐₓ = 26 900

| - Analyse élémentaire pour C₃₀ H₅₂ O₆ Si₃ | | | |
|---|---|---|---|
| théorie | C60.76 | H8.84 | Si14.21 |
| trouvé | C60.54 | H8.87 | Si13.96 |

### EXEMPLE 5 :

### Préparation du dérivé de formule (10) :

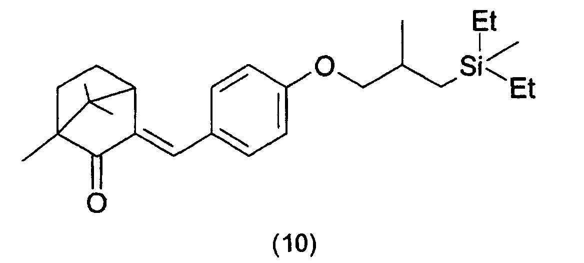

A une solution de 1,7,7-Triméthyl-3-[4-(2-méthyl-allyloxy)-benzylidène]-bicyclo[2.2.1] heptan-2-one (15,5 g, 0,05 mole) et de catalyseur (complexe à 3-3.5% poids de Pt dans du cyclovinylméthylsiloxane de Hüls Petrarch PC085 : 200 µl) dans 10 ml de toluène sec porté à 70°C, on ajoute goutte à goutte en 20 minutes 5,37 g (0,0525 mole) de diéthyl méthyl silane. On laisse à cette température pendant 48 heures. On concentre le mélange réactionnel et on obtient après chromatographie sur silice (éluant : heptane/ dichlorométhane :) 11,8 g
(Rendement : 57 %) d'une huile visqueuse incolore du dérivé de l'exemple 5 qui cristallise :
- Pf : 39-40°C
- UV (Ethanol) λₘₐₓ = 320 nm, εₘₐₓ = 28 230

| - Analyse élémentaire pour C₂₆ H₄₀ O₂ Si | | | |
|---|---|---|---|
| théorie | C75.67 | H9.77 | Si6.81 |
| trouvé | C75.42 | H9.77 | Si7.10 |

### EXEMPLE 6 :

### Préparation du dérivé de formule (11):

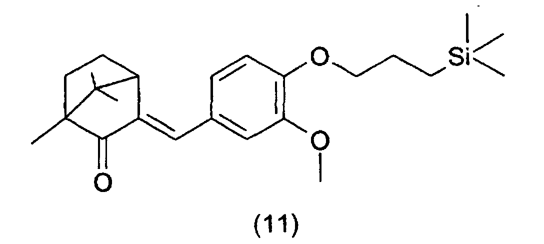

a) Première étape : préparation du 3-méthoxy-4-(3-triméthylsilanyl-propyloxy)-benzaldéhyde :
Dans un mélange de vanilline (30,4 g, 0,2 mole) et de carbonate de potassium (30,4 g, 0,22 mole) dans 150 ml de DMF sec porté à 80°C sous azote, on ajoute goutte à goutte en 20 minutes du 3-chloropropyltriméthylsilane (33,14 g, 0,22 mole). On laisse 4 heures à 95-110°C. On refroidit et verse le mélange réactionnel dans de l'eau glacée. La phase aqueuse est extraite 3 fois au dichlorométhane. Les phases organiques sont séchées sur sulfate de sodium et concentrées sous vide. Après distillation sous vide 5,32 Pa (0,04 mmHg), on obtient 47,5g (Rendement : 89 %) de 3-méthoxy-4-(3-triméthylsilanyl-propyloxy)-benzaldéhyde sous forme d'une huile légèrement rosée distillant à 112-114°C et utilisée telle quelle dans l'étape suivante.
b) Deuxième étape : préparation du dérivé de l'exemple 6
On chauffe à 80°C pendant 30 minutes un mélange de camphre (8,36 g, 0,055 mole) et de NaH à 50 % dans l'huile (2,64 g, 0,055 mole; rincé à l'heptane sec puis au diméthoxy éthane sec) dans 40 ml de diméthoxy éthane sec. On y ajoute goutte à goutte en 20 minutes à 80°C le dérivé précédent (13,32 g, 0,05 mole) dissout dans 30 ml de diméthoxy éthane. On laisse sous agitation à 80°C pendant 3 heures. Après refroidissement, le mélange est versé dans de l'eau glacée. Le précipité obtenu est lavé à l'eau puis recristallisé dans l'éthanol pour donner 12,4 g (Rendement : 62 %) du dérivé de l'exemple 6 sous forme d'une poudre blanche:
- Pf : 79-80°C
- UV (Ethanol) λₘₐₓ = 331 nm, εₘₐₓ = 21 300

| - Analyse élémentaire pour C₂₄ H₃₆ O₃ Si | | | |
|---|---|---|---|
| théorie | C71.95 | H9.06 | Si7.01 |
| trouvé | C71.80 | H9.05 | Si6.80 |

### EXEMPLE 7 :

### Préparation du dérivé de formule (12) :

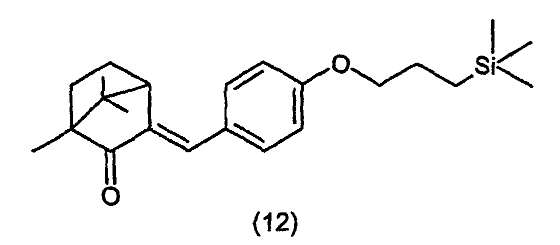

a) Première étape : préparation du 4-(3-triméthylsilanyl-propyloxy)-benzaldéhyde
Dans un mélange de 4-hydroxy benzaldéhyde (24,4 g, 0,2 mole) et de carbonate de potassium (30,4 g, 0,22 mole) dans 150 ml de DMF sec porté à 120°C sous azote, on ajoute goutte à goutte en 10 minutes du 3-chloropropyltrlméthylsilane (33,14 g, 0,22 mole). On laisse 2 heures 30 minutes à 120-130°C. On refroidit et verse le mélange réactionnel dans de l'eau glacée. La phase aqueuse est extraite 3 fois au dichlorométhane. Les phases organiques sont séchées sur sulfate de sodium et concentrées sous vide. Après distillation sous vide 26,64 Pa (0,2 mmHg), on obtient 40,5g (Rendement : 86 %) de 4-(3-triméthylsilanyl-propyloxy)-benzaldéhyde sous forme d'une huile incolore distillant à 110-114°C et utilisée telle quelle dans l'étape suivante.
b) Deuxième étape : préparation du dérivé de l'exemple 7
On chauffe à 80°C pendant 30 minutes un mélange de camphre (15,2 g, 0,1 mole) et de NaH à 50 % dans l'huile (4,8 g, 0,1 mole; rincé à l'heptane sec puis au diméthoxy éthane sec) dans 50 ml de diméthoxy éthane sec. On y ajoute goutte à goutte en 20 minutes à 80°C le dérivé précédent (21,3 g, 0,09 mole) dissout dans 30 ml de diéthoxy éthane. On laisse sous agitation à 80°C pendant 2 heures. Après refroidissement, le mélange est versé dans de l'eau glacée. Le précipité obtenu est lavé à l'eau puis recristallisé dans l'éthanol pour donner 20,6 g (Rendement : 60 %) du dérivé de l'exemple 7 sous forme d'une poudre blanche :
- Pf : 100-101°C
- UV (Ethanol) λₘₐₓ = 320 nm, εₘₐₓ = 26 530

| - Analyse élémentaire pour C₂₃ H₃₄ O₂ Si | | | |
|---|---|---|---|
| théorie | C74.54 | H9.25 | Si7.58 |
| trouvé | C74.62 | H9.17 | Si7.80 |

### EXEMPLE 8 :

On a réalisé les compositions suivantes (les quantités sont exprimées en poids % par rapport au poids total de la composition) :

| Composition A (comparative) : | |
|---|---|
| **Ingrédients** | **Composition A** |
| Myristate d'isopropyle | 30 |
| ®Parsol 1789 | 1,5 |
| Ethanol | qsp 100 |

| Composition A' (comparative) : | |
|---|---|
| **Ingrédients** | **Composition A'** |
| Myristate d'isopropyle | 30 |
| 3- (4-méthylbenzylidène)-camphre (®Eusolex 6300) | 5 |
| ®Parsol 1789 | 1,5 |
| Ethanol | qsp 100 |

Les compositions B à E (invention) comprennent en outre 5% en poids de composé benzylidène camphre siliconé selon l'invention :

| **Ingrédients** | **Composition B** | **Composition C** | **Composition D** | **Composition E** |
|---|---|---|---|---|
| Myristate d'isopropyle | 30 | 30 | 30 | 30 |
| ®Parsol 1789 | 1,5 | 1,5 | 1,5 | 1,5 |
| Benzylidène camphre siliconé selon l'invention | **exemple 5** | **exemple 6** | **exemple 7** | **exemple 2** |
| Ethanol | qsp 100 | qsp 100 | qsp 100 | qsp 100 |

Pour chacune de ces compositions, on a déterminé le pourcentage de ®Parsol 1789 (4-tert-butyl-4'-méthoxydibenzoylméthane) résiduel après Irradiation par des UV selon le protocole suivant : pour chaque formule, on a préparé trois échantillons témoins et trois échantillons tests. On a étalé à la main 2µl/cm² de formule sur des plaques en verre dépoli. Puis on a exposé les plaques dans une enceinte dont la température est régulée aux environs de 35-40 °C (®SUNTEST CPS Heraeus), pendant le temps nécessaire à obtenir une exposition, exprimée par rapport au flux UVA, de 30 J/cm², afin de simuler une irradiation UV naturelle en conservant les plaques témoins à l'obscurité pendant le temps d'irradiation des autres plaques.

On a ensuite dosé les échantillons de la manière suivante : on a procédé à l'extraction des filtres en immergeant chaque plaque dans 50 g d'éthanol afin de solubiliser les filtres. Les plaques et le solvant contenant les filtres ont ensuite été traités aux ultrasons pendant 15 minutes pour assurer une extraction efficace. Les solutions obtenues sont analysées par spectrophotométrie au λₘₐₓ de chacun des filtres.

Pour chaque formule testée, le taux de 4-tert-butyl-4'-méthoxydibenzoylméthane résiduel après irradiation est donné par le rapport de sa concentration dans l'échantillon irradié à sa concentration dans l'échantillon non irradié.

Les résultats en pourcentage de 4-tert-butyl-4'-méthoxydibenzoylméthane restant sont consignés dans le tableau (I) suivant :

**Tableau (I)**

| Composition | % de DO résiduelle Parsol 1789 |
|---|---|
| Composition A (comparative) | 5 ± 1 |
| Composition A' (comparative) | 58,8 |
| Composition B (invention) | 76 ± 6 |
| Composition C (invention) | 81 ± 6 |
| Composition D (invention) | 79 ± 7 |
| Composition E (invention) | 69 ± 3 |

Ces résultats montrent clairement que la présence d'un composé silanique ou organosiloxane à groupement benzylidène camphre améliore la photostabilité du 4-tert-butyl-4'-méthoxydibenzoylméthane.

Ces résultats montrent également que la photostabilité du 4-tert-butyl-4'-méthoxydibenzoylméthane obtenue en présence d'un composé benzylidène camphre hydrocarboné est encore améliorée en utilisant à la place de ce dernier des composés silaniques ou organosiloxanes à groupement benzylidène camphre.

## Revendications

1. Procédé pour améliorer la stabilité d'au moins un dérivé du dibenzoylméthane vis-à-vis du rayonnement UV, caractérisé par le fait qu'il consiste à associer au dit dérivé du dibenzoylméthane une quantité efficace d'un composé silanique ou organosiloxanique à fonction benzylidène camphre.

2. Procédé selon la revendication 1, caractérisé par le fait composé silanique ou organosilanique à fonction benzylidène camphre répond à la formule (I) suivante : 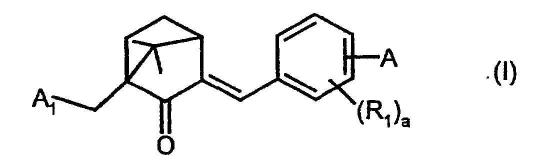 dans laquelle :
- R₁, identiques ou différents, représentent l'hydrogène, un radical OH, un radical alkyle saturé ou non, linéaire ou ramifié, en C₁-C₁₀, un radical alcoxy linéaire ou ramifié en C₁-C₁₀ ou -OSi(CH₃)₃, deux R₁ adjacents pouvant former ensemble un groupement alkylidène dioxy dans lequel le groupe alkylidène contient de 1 à 2 atomes de carbone ;
- a est un nombre entier allant de 1 à 3;
- A est l'hydrogène ou un radical de formule -L-W tel que :
- L est un radical divalent de formules (IIa) ou (IIb) suivantes :
-CH₂-CHR₂-[C(R₃)₂]ₘ-(CO)ₙ-(O)ₒ-(Z)ₚ-(Y)_{q}- (IIa)
-CH=CR₂-[C(R₃)₂]ₘ-(CO)ₙ-(O)ₒ-(Z)ₚ-(Y)_{q}- (IIb)
dans laquelle :
- R₂ et R₃, identiques ou différents, représentent l'hydrogène ou un radical alkyle linéaire ou ramifié en C₁-C₅,
- Z est un radical di-yle en C₁-C₆, linéaire ou ramifié, saturé ou insaturé, éventuellement substitué par un radical hydroxyle ou alcoyle en C₂-C₈, linéaire ou ramifié, saturé ou insaturé,
- Y représente -O-, -NR₄-, -SO₂NH-, -(CO)O-, -(CO)NH- ou -O(CO)NH-, avec R₄ qui représente l'hydrogène ou un radical alkyle en C₁-C₅ ;
- m est un nombre entier allant de 0 à 10 ;
- n est 0 ou 1 ;
- o est 0 ou 1 ;
- p est 0 ou 1 ;
- q est 0 ou 1 ;
- A₁ est l'hydrogène ou le radical -L₁-W dans lequel le radical L₁ a la définition de L sous réserve que lorsque q = 1 alors Y représente -SO₂NH- et étant entendu qu'un seul des deux radicaux A et A₁ est l'hydrogène ;
- W est un radical de formules (1), (2) ou (3) suivantes : 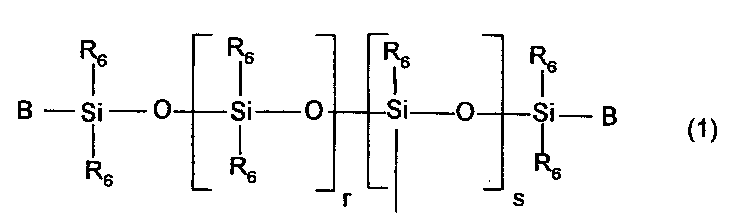 ou 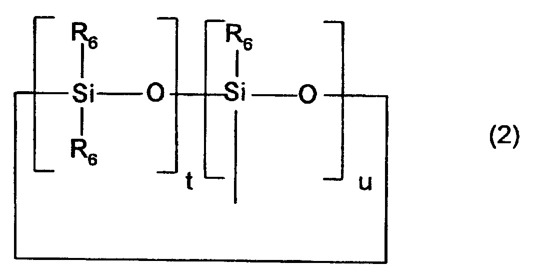
-Si(R₆)₃ (3)
dans lesquelles :
- R₆, identiques ou différents, sont choisis parmi les radicaux alkyles linéaires ou ramifiés en C₁-C₃₀, phényle, au moins 80% en nombre des radicaux R₆ étant méthyle ;
- B, identiques ou différents sont choisis parmi les radicaux R₆ et le radical X de formule suivante : 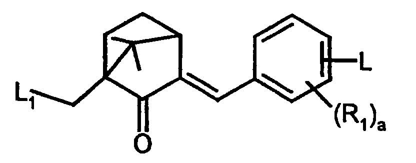 dans laquelle R₁, L, L₁ et a ont les mêmes significations que ci-dessus ;
- r est un nombre entier compris entre 0 et 200 inclusivement, et s est un nombre entier compris entre 0 et 50 inclusivement, et si s = 0, au moins l'un des deux symboles B désigne X ;
- u est un nombre entier compris entre 1 et 10 inclusivement, et t est un nombre entier compris entre 0 et 10 inclusivement, étant entendu que t + u est égal ou supérieur à 3.

3. Procédé selon la revendication 2, caractérisé par le fait que le composé de formule (I) répond au moins à l'une des caractéristiques suivantes :
- R₁ = H, OCH₃, CH₃ ou deux radicaux R₁ adjacents forment un méthylène dioxy,
- R₂ = H ou CH₃;
- n = 0;
- q = 1.

4. Procédé selon la revendication 2, caractérisé par le fait que le composé de formule (I) répond en outre à au moins à l'une des caractéristiques suivantes :
- R₆ est méthyle ;
- B est méthyle ;
- r est compris entre 5 et 20 inclus ;
- s est compris entre 2 et 15 inclus;
- t+u est compris entre 3 et 10 inclus ;
- m = 1 ;
- R₃ est l'hydrogène ou un radical méthyle.

5. Procédé selon la revendication 2, caractérisé par le fait que le composé de formule (I) est choisi parmi ceux où s = 0.

6. Procédé selon la revendication 2, caractérisé par le fait que le composé de formule (I) est choisi parmi les composés de formules suivantes :

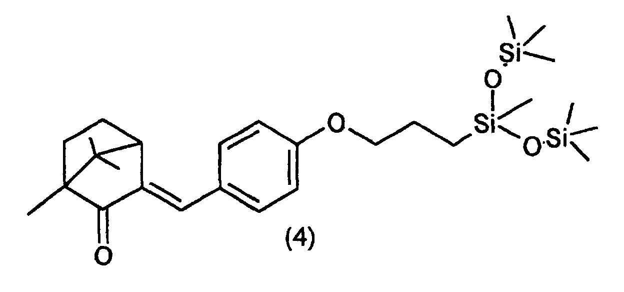

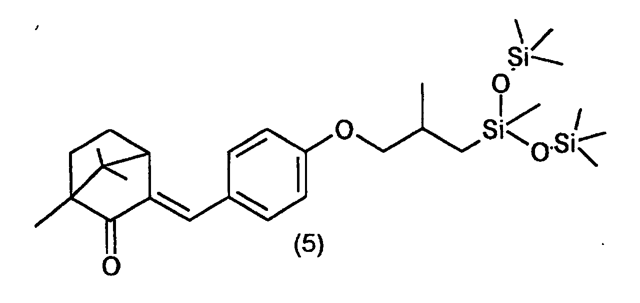

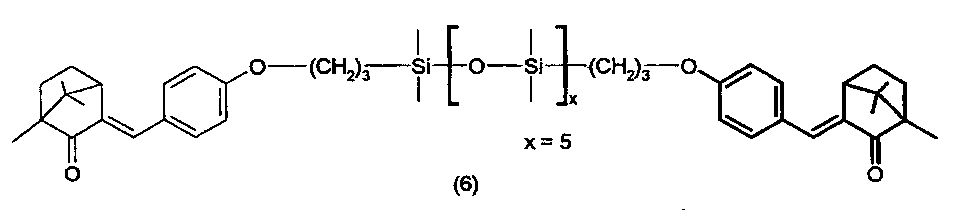

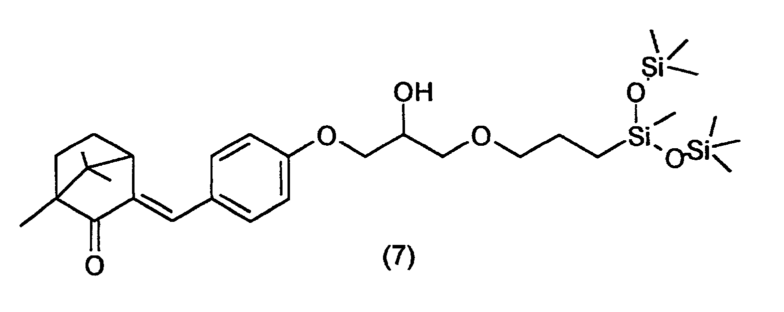

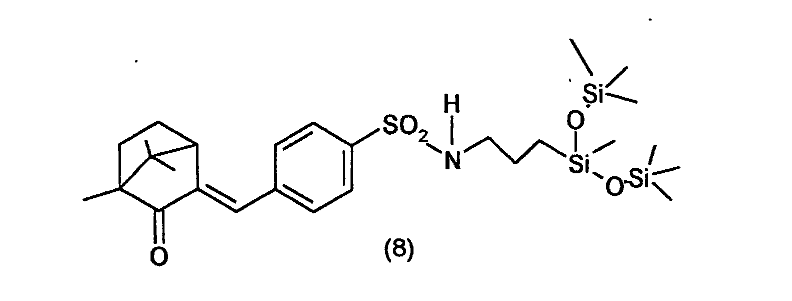

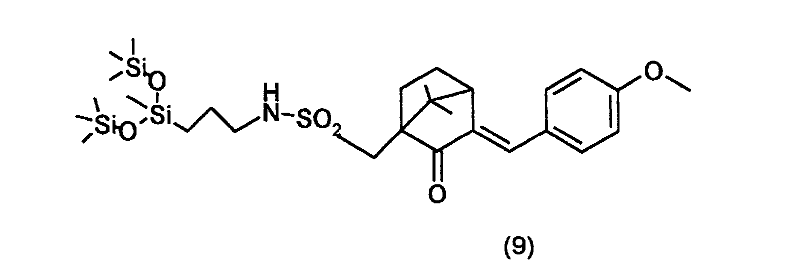

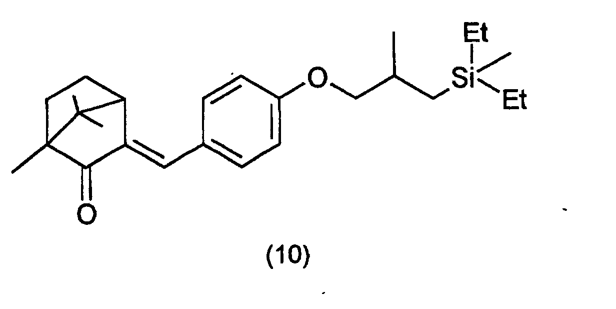

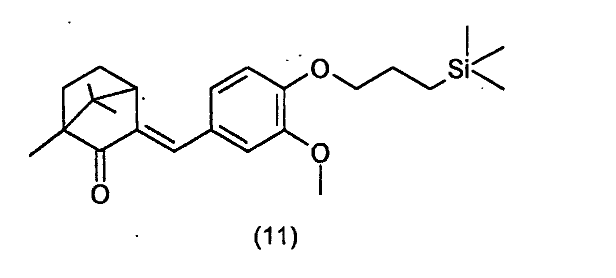

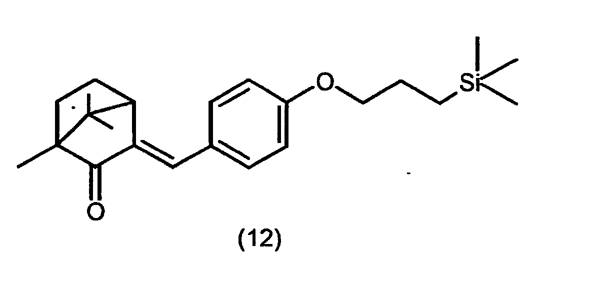

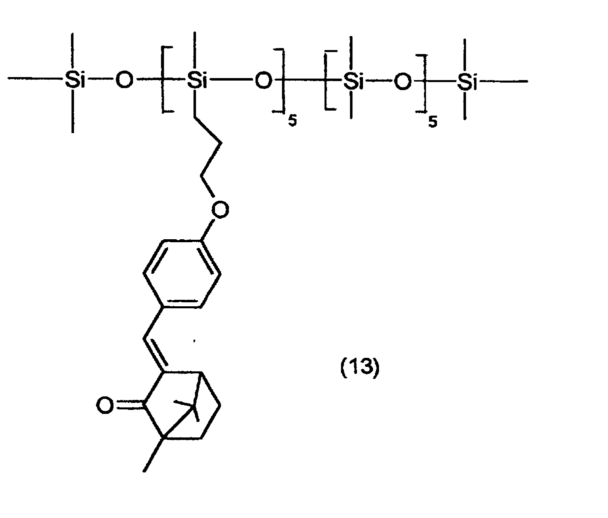

7. Procédé selon la revendication 2, caractérisé par le fait que le composé de formule (I) est choisi parmi ceux où p est égal à 1 et le radical Z est un radical di-yle en C₁-C₆ linéaire ou ramifié, saturé ou insaturé et possédant un radical hydroxyle.

8. Procédé selon la revendication 7 où le composé, de formule (I) est le composé de structure :

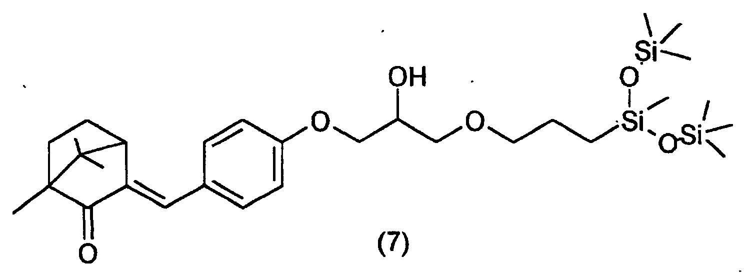

9. Procédé selon la revendication 8 où le composé de formule (I) est choisi dans le groupe constitué par les composés suivants :

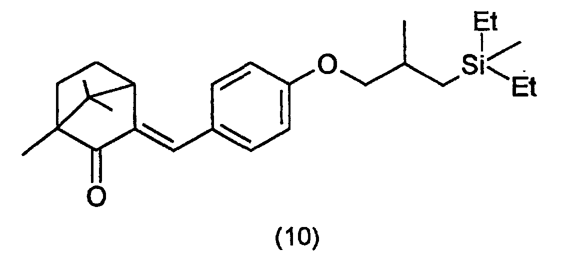

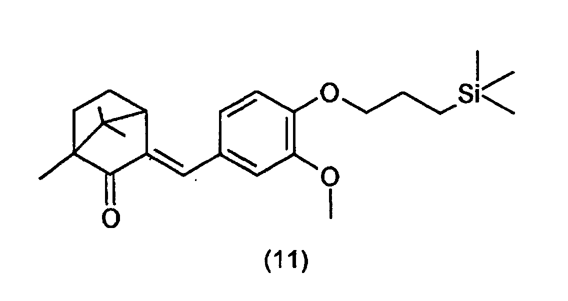

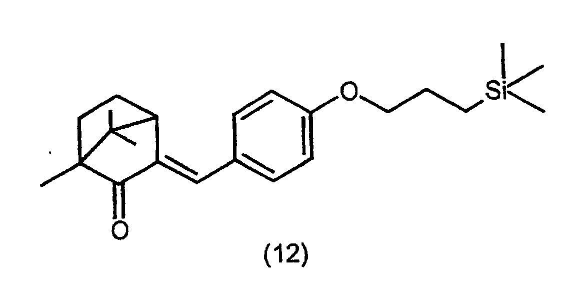

10. Procédé selon l'une quelconque des revendications 1 à 9, caractérisé par le fait que le dérivé de dibenzoylméthane est choisi parmi :
- le 2-méthyldibenzoylméthane
- le 4-méthyldibenzoylméthane
- le 4-isopropyldibenzoylméthane
- le 4-tert-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

11. Procédé selon la revendication 10, caractérisé par le fait que le dérivé de dibenzoylméthane est le 4-(ter.-butyl) 4'-méthoxy dibenzoylméthane.

12. Procédé selon la revendication 10, caractérisé par le fait que le dérivé de dibenzoylméthane est le 4-isopropyl-dibenzoylméthane.

13. Composition cosmétique filtrante photostable pour la photoprotection par voie topique de la peau et/ou des cheveux contre le rayonnement ultraviolet, en particulier le rayonnement solaire, du type comprenant, dans un support cosmétiquement acceptable, au moins un dérivé du dibenzoylméthane, caractérisées par le fait qu'elle comprend en outre une quantité efficace d'au moins un composé silanique ou organosiloxanique à fonction benzylidène camphre.

14. Composition selon la revendication 13, caractérisée par le fait que caractérisé par le fait que le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopmpyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl4-tert.-butyl-4'-méthoxydibenzoylméthane.

15. Composition selon la revendication 14, caractérisé par le fait que le dérivé de dibenzoylméthane est le 4-(ter.-butyl) 4'-méthoxy dibenzoylméthane.

16. Composition selon la revendication 14, caractérisé par le fait que le dérivé de dibenzoylméthane est le 4-isopropyl-dibenzoylméthane.

17. Composition selon l'une quelconque des revendications 14 à 17, caractérisées par le fait que la teneur en dérivé(s) du dibenzoylméthane est comprise entre 0,01 % et 10 % en poids par rapport au poids total de la composition.

18. Composition selon la revendication 17, caractérisée par le fait que ladite teneur est comprise entre 0,1 % et 6 % en poids par rapport au poids total de la composition.

19. Composition selon l'une quelconque des revendications 13 à 18, où le composé silanique ou organosiloxanique à fonction benzylidène camphre répond à la formule (I) suivante : 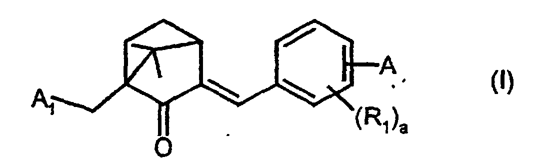 dans laquelle:
- R₁, identiques ou différents, représentent l'hydrogène, un radical OH, un radical alkyle saturé ou non, linéaire ou ramifié, en C₁-C₁₀, un radical alcoxy linéaire ou ramifié en C₁-C₁₀ ou -OSi(CH₃)₃, deux R₁ adjacents pouvant former ensemble un groupement alkylidène dioxy dans lequel le groupe alkylidène contient de 1 à 2 atomes de carbone ;
- a est un nombre entier allant de 1 à 3 ;
- A est l'hydrogène ou un radical de formule -L-W tel que :
- L est un radical divalent de formules (IIa) ou (IIb) suivantes:
-CH₂-CHR₂-[C(R₃)₂]ₘ-(CO)ₙ-(O)ₒ-(Z)ₚ-(Y)_{q}- (IIa)
-CH=CR₂-[C(R₃)₂]ₘ-(CO)ₙ-(O)ₒ-(Z)ₚ-(Y)_{q}- (IIb)
dans laquelle:
- R₂ et R₃, identiques ou différents, représentent l'hydrogène ou un radical alkyle linéaire ou ramifié en C₁-C₅,
- Z est un radical di-yle en C₁-C₆, linéaire ou ramifié, saturé ou insaturé, éventuellement substitué par un radical hydroxyle ou alcoyle en C₂-C₈, linéaire ou ramifié, saturé ou insaturé,
- Y représente -O-, -NR₄-, -SO₂NH-, -(CO)O-, -(CO)NH- ou -O(CO)NH-, avec R₄ qui représente l'hydrogène ou un radical alkyle en C₁-C₅ ;
- m est un nombre entier allant de 0 à 10 ;
- n est 0 ou 1 ;
- o est 0 ou 1 ;
- p est 0 ou 1 ;
- q est 0 ou 1 ;
- A₁ est l'hydrogène ou le radical -L₁-W dans lequel le radical L₁ a la définition de L sous réserve que lorsque q = 1 alors Y représente -SO₂NH- et étant entendu qu'un seul des deux radicaux A et A₁ est l'hydrogène ;
- W est un radical de formules (1), (2) ou (3) suivantes: 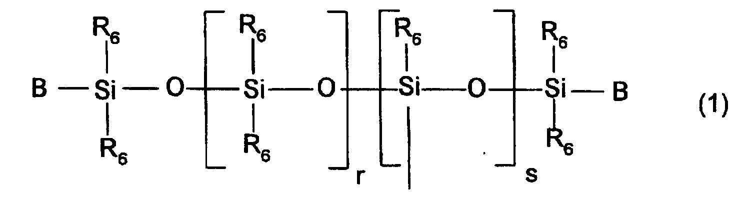 ou 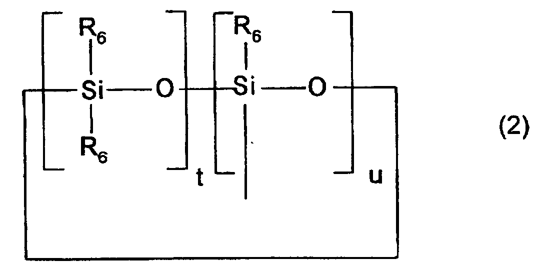
-Si(R₆)₃ (3)
dans lesquelles :
- R₆, identiques ou différents, sont choisis parmi les radicaux alkyles linéaires ou ramifiés en C₁-C₃₀, phényle, au moins 80% en nombre des radicaux R₆ étant méthyle ;
- B, identiques ou différents sont choisis parmi les radicaux R₆ et le radical X de formule suivante : 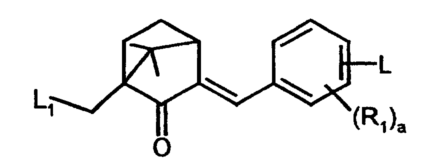 dans laquelle R₁, L, L₁ et a ont les mêmes significations que ci-dessus ;
- r est un nombre entier compris entre 0 et 200 inclusivement, et s est un nombre entier compris entre 0 et 50 inclusivement, et si s = 0, au moins l'un des deux symboles B désigne X ;
- u est un nombre entier compris entre 1 et 10 inclusivement, et t est un nombre entier compris entre 0 et 10 inclusivement, étant entendu que t + u est égal ou supérieur à 3.

20. Composition selon la revendication 19, caractérisée par le fait que le composé de formule (I) répond au moins à l'une des caractéristiques suivantes :
- R₁ = H, OCH₃, CH₃ ou deux radicaux R₁ adjacents forment un méthylène dioxy,
- R₂ = H ou CH₃ ;
- n = 0 ;
- q = 1.

21. Composition selon la revendication 19, caractérisée par le fait que le composé de formule (I) répond en outre à au moins à l'une des caractéristiques suivantes :
- R₆ est méthyle ;
- B est méthyle ;
- r est compris entre 5 et 20 inclus ;
- s est compris entre 2 et 15 inclus ;
- t+u est compris entre 3 et 10 inclus ;
- m = 1 ;
- R₃ est l'hydrogène ou un radical méthyle.

22. Composition selon la revendication 19, caractérisée par le fait que le composé de formule (I) est choisi parmi ceux où s = 0.

23. Composition selon la revendication 19, caractérisé par le fait que le composé de formule (I) est choisi parmi les composés de formules suivantes:

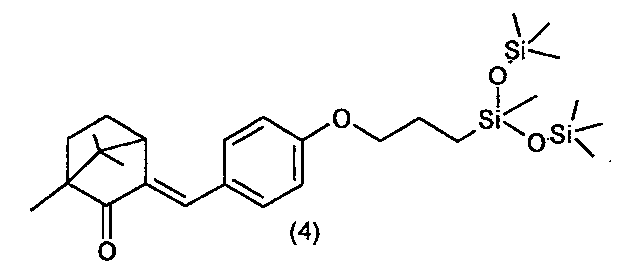

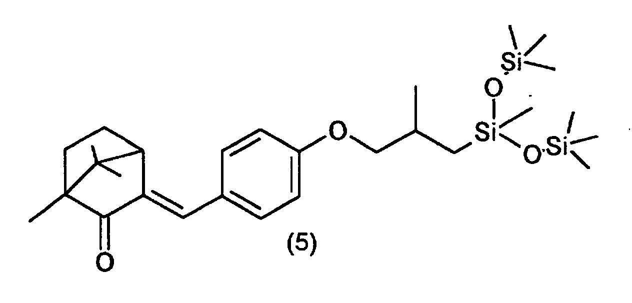

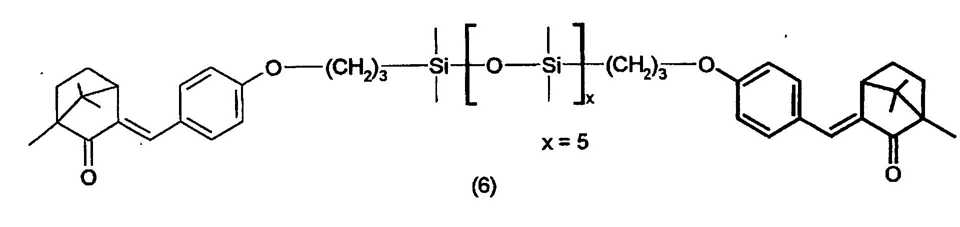

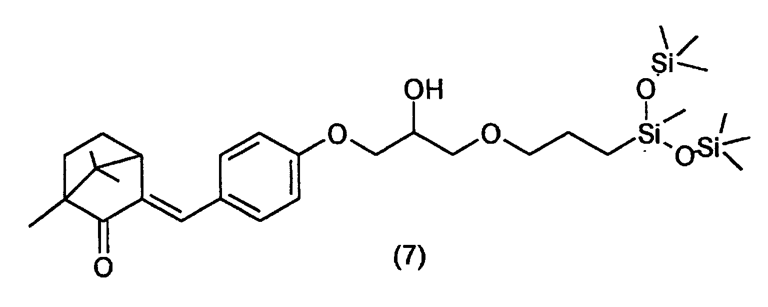

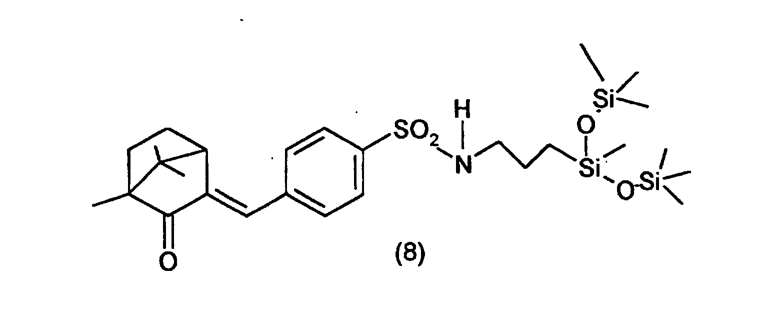

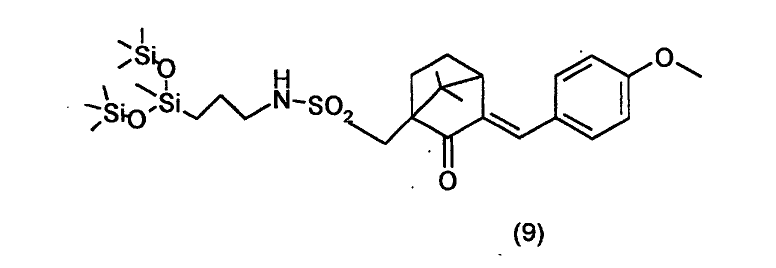

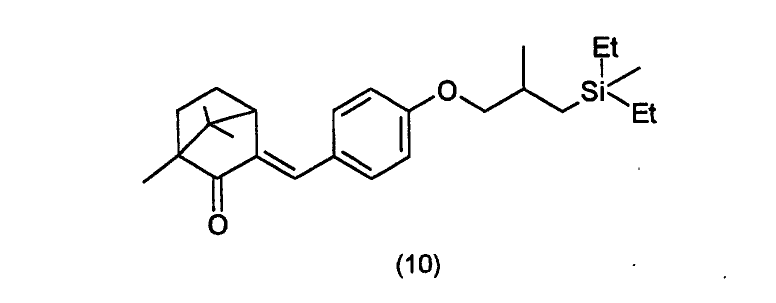

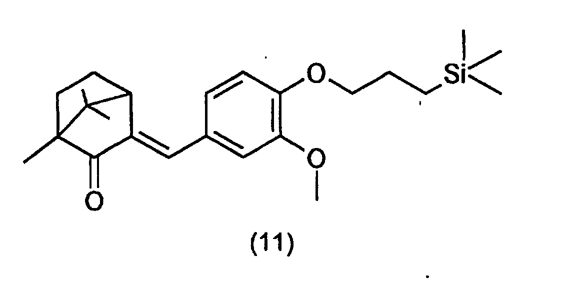

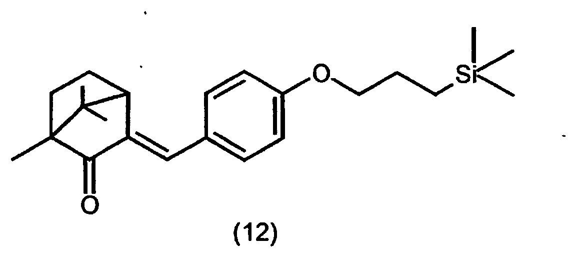

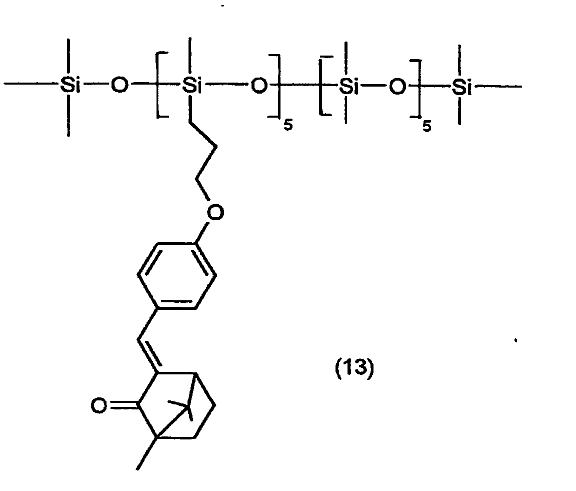

24. Composition selon la revendication 19, caractérisée par le fait que le composé de formule (I) est choisi parmi ceux où p est égal à 1 et le radical Z est un radical di-yle en C₁-C₆ linéaire ou ramifié, saturé ou insaturé et possédant un radical hydroxyle.

25. Composition selon l'une quelconque des revendications 13 à 24, caractérisée par le fait que la teneur en composé(s) silanique ou organosiloxanique(s) est au moins égale à 0,5 % en poids, par rapport au poids total de la composition.

26. Composition selon la revendication 25, caractérisée par le fait que ladite teneur va de 0,5 % à 20 % en poids, par rapport au poids total de la composition.

27. Composé répondant à la formule (I) suivante : 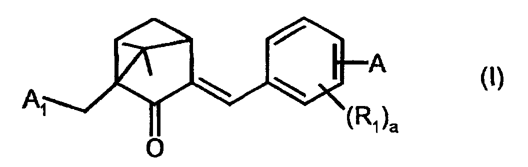 dans laquelle :
- R₁, identiques ou différents, représentent l'hydrogène, un radical OH, un radical alkyle saturé ou non, linéaire ou ramifié, en C₁-C₁₀, un radical alcoxy linéaire ou ramifié en C₁-C₁₀ ou -OSi(CH₃)₃, deux R₁ adjacents pouvant former ensemble un groupement alkylidène dioxy dans lequel le groupe alkylidène contient de 1 à 2 atomes de carbone;
- a est un nombre entier allant de 1 à 3 ;
- A est l'hydrogène ou un radical de formule -L-W tel que :
- L est un radical divalent de formules (IIa) ou (IIb) suivantes :
-CH₂-CHR₂-[C(R₃)₂]ₘ-(CO)ₙ-(O)ₒ-Z-(Y)_{q}- (IIa)
-CH=CR₂-[C(R₃)₂]ₘ-(CO)ₙ-(O)ₒ-Z-(Y)_{q}- (IIb)
dans laquelle :
- R₂ et R₃, identiques ou différents, représentent l'hydrogène ou un radical alkyle linéaire ou ramifié en C₁-C₅,
- Z est un radical di-yle en C₁-C₆ linéaire ou ramifié, saturé ou insaturé et possédant un radical hydroxyle ;
- Y représente -O-, -NR₄-, -SO₂NH-, -(CO)O-, -(CO)NH- ou -O(CO)NH-, avec R₄ qui représente l'hydrogène ou un radical alkyle en C₁-C₅ ;
- m est un nombre entier allant de 0 à 10 ;
- n est 0 ou 1 ;
- o est 0 ou 1 ;
- q est 0 ou 1 ;
- A₁ est l'hydrogène ou le radical -L₁-W dans lequel le radical L₁ a la définition de L sous réserve que lorsque q = 1 alors Y représente -SO₂NH- et étant entendu qu'un seul des deux radicaux A et A₁ est l'hydrogène ;
- W est un radical de formules (1), (2) ou (3) suivantes : 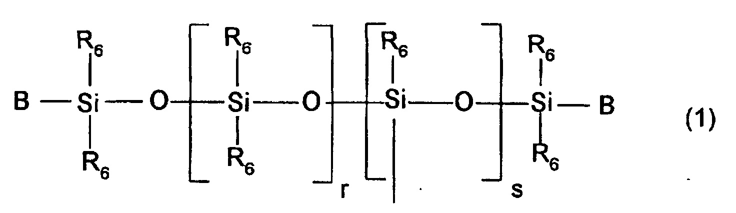 ou 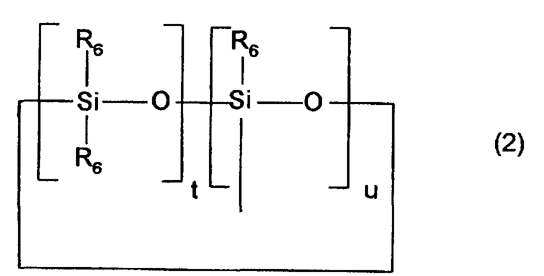
-Si(R₆)₃ (3)
dans lesquelles :
- R₆, identiques ou différents, sont choisis parmi les radicaux alkyles linéaires ou ramifiés en C₁-C₃₀, phényle, au moins 80% en nombre des radicaux R₆ étant méthyle ;
- B, identiques ou différents sont choisis parmi les radicaux R₆ et le radical X de formule suivante : 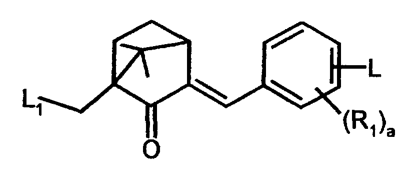 dans laquelle R₁, L, L₁ et a ont les mêmes significations que ci-dessus ;
- r est un nombre entier compris entre 0 et 200 inclusivement, et s est un nombre entier compris entre 0 et 50 inclusivement, et si s = 0, au moins l'un des deux symboles B désigne X ;
- u est un nombre entier compris entre 1 et 10 inclusivement, et t est un nombre entier compris entre 0 et 10 inclusivement, étant entendu que t + u est égal ou supérieur à 3.

28. Composé de structure :

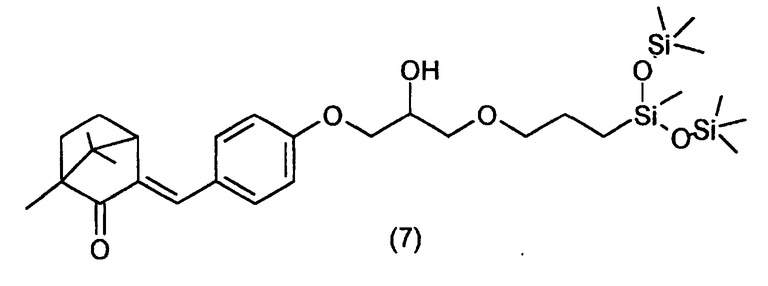

29. Composé de structure :

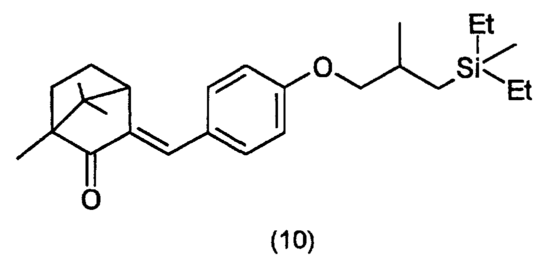

30. Composé de structure :

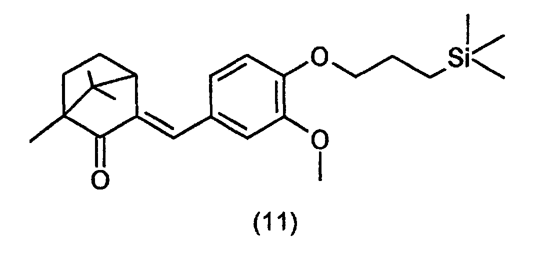

31. Composé de structure :

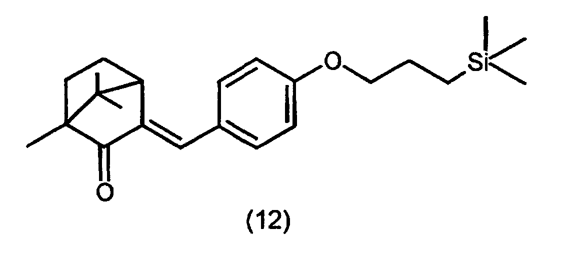

32. Utilisation d'un composé silanique ou organosiloxanique à fonction benzylidène camphre tel que défini selon l'une quelconque des revendications 1 à 9, 27 à 31, dans la préparation d'une composition cosmétique ou dermatologique contenant au moins un dérivé du dibenzoylméthane, pour améliorer la stabilité dudit dérivé du dibenzoylméthane vis-à-vis du rayonnement UV.

## Patentansprüche

1. Verfahren zur Verbesserung der Stabilität mindestens eines Dibenzoylmethanderivats gegenüber UV-Strahlung, dadurch gekennzeichnet, daß es darin besteht, das Dibenzoylmethanderivat mit einer wirksamen Menge einer Silan- oder Organosiloxanverbindung mit Benzylidencamphergruppe zu kombinieren.

2. Verfahren nach Anspruch 1, dadurch gekennzeichnet, daß die Silan- oder Organosiloxanverbindung mit Benzylidencamphergruppe der folgenden Formel (I) entspricht: 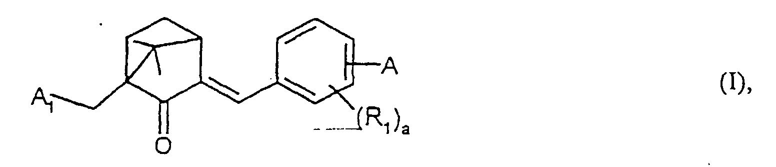 worin bedeuten:
- die Gruppen R₁, die identisch oder voneinander verschieden sind, Wasserstoff, die Gruppe OH, eine gesättigte oder ungesättigte, geradkettige oder verzweigte C₁₋₁₀-Alkylgruppe, eine geradkettige oder verzweigte C₁₋₁₀-Alkoxygruppe oder -OSi(CH₃)₃, wobei zwei angrenzende Gruppen R₁ eine Alkylidendioxygruppe bilden können, worin die Alkylidengruppe 1 bis 2 Kohlenstoffatome aufweist;
- a eine ganze Zahl von 1 bis 3;
- A Wasserstoff oder eine Gruppe -L-W:
- L eine zweiwertige Gruppe der folgenden Formeln (IIa) oder (IIb):
-CH₂-CHR₂-[C(R₃)₂]ₘ-(CO)ₙ-(O)ₒ-(Z)ₚ-(Y)_{q}- (IIa)
-CH=CR₂-[C(R₃)₂]ₘ-(CO)ₙ-(O)ₒ-(Z)ₚ-(Y)_{q}- (IIb)
worin bedeuten:
- R₂ und R₃, die identisch oder voneinander verschieden sind, Wasserstoff oder eine geradkettige oder verzweigte C₁₋₅-Alkylgruppe,
- Z eine geradkettige oder verzweigte, gesättigte oder ungesättigte, gegebenenfalls mit einer Hydroxygruppe oder einer geradkettigen oder verzweigten, gesättigten oder ungesättigten C₂₋₈-Alkylgruppe substituierte C₁₋₆-Diylgruppe,
- Y eine Gruppe -O-, -NR₄-, -SO₂NH-, -(CO)O-, -(CO)NH- oder -O(CO)NH-, wobei R₄ Wasserstoff oder eine C₁₋₅-Alkylgruppe bedeutet,
- m Null oder eine ganze Zahl von 1 bis 10,
- n 0 oder 1,
- o 0 oder 1,
- p 0 oder 1,
- q 0 oder 1,
- A₁ Wasserstoff oder eine Gruppe -L₁-W, wobei L₁ der Definition von L entspricht, mit der Maßgabe, daß Y -SO₂NH- bedeutet, wenn q = 1;
- mit der Maßgabe, daß nur eine der beiden Gruppen A und A₁ Wasserstoff bedeutet;
- W eine Gruppe der folgenden Formeln (1), (2) oder (3): 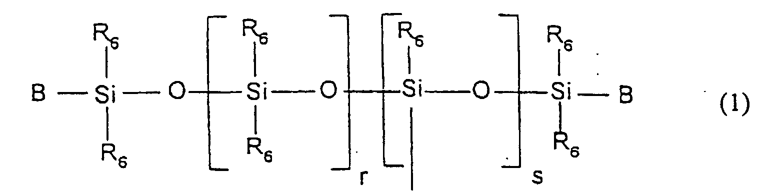 oder 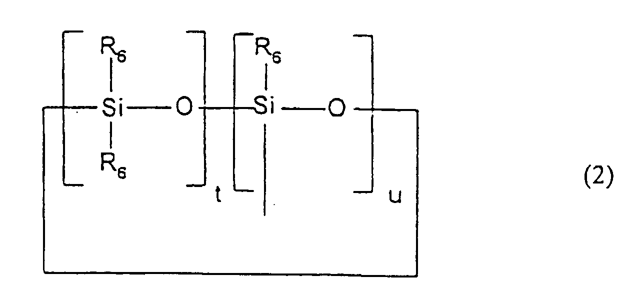 oder
Si(R₆)₃ (3),
worin:
- die Gruppen R₆, die identisch oder voneinander verschieden sind, unter den geradkettigen oder verzweigten C₁₋₃₀-Alkylgruppen und Phenyl ausgewählt sind, wobei mindestens 80 % der Gruppen R₆ Methyl bedeuten,
- die Gruppen B, die identisch oder voneinander verschieden sind, unter den Gruppen R₆ und der Gruppe X der folgenden Formel ausgewählt sind: 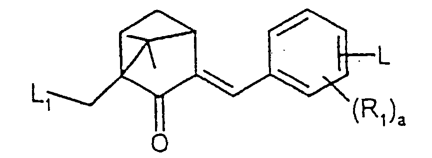 wobei R₁, L, L₁ und a die oben angegebenen Bedeutungen aufweisen;
- r 0 oder eine ganze Zahl im Bereich von 1 bis 200 und s 0 oder eine ganze Zahl im Bereich von 1 bis 50 bedeutet, und mindestens eine der beiden Gruppen B X bedeutet, wenn s = 0,
- u eine ganze Zahl im Bereich von 1 bis 10 und t 0 oder eine ganze Zahl im Bereich von 1 bis 10 ist, mit der Maßgabe, daß t + u 3 bedeutet oder darüber liegt.

3. Verfahren nach Anspruch 2, dadurch gekennzeichnet, daß die Verbindung der Formel (I) mindestens eine der folgenden Eigenschaften aufweist:
- R₆ = Methyl,
- B = Methyl.
- r liegt im Bereich von 5 bis 20,
- s liegt im Bereich von 2 bis 15,
- t + u liegt im Bereich von 3 bis 10,
- m = 1,
- R₃ = Wasserstoff oder Methyl.

4. Verfahren nach Anspruch 2, dadurch gekennzeichnet, daß die Verbindung der Formel (I) unter den Verbindungen mit s = 0 ausgewählt ist.

5. Verfahren nach Anspruch 2, dadurch gekennzeichnet, daß die Verbindung der Formel (I) unter den Verbindungen der folgenden Formeln ausgewählt ist:

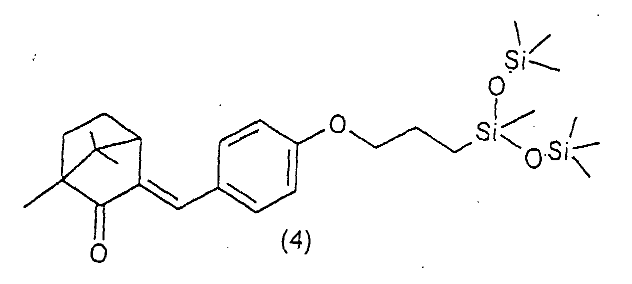

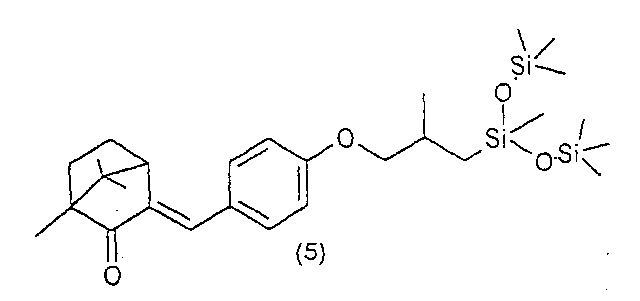

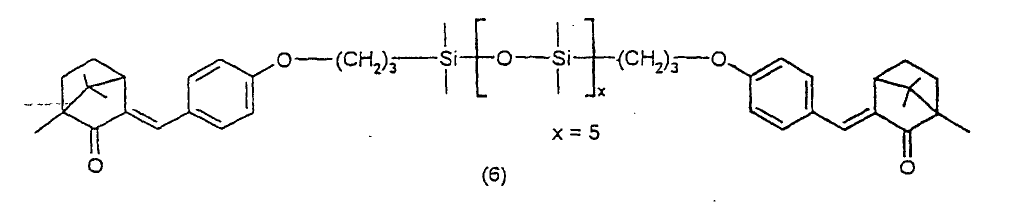

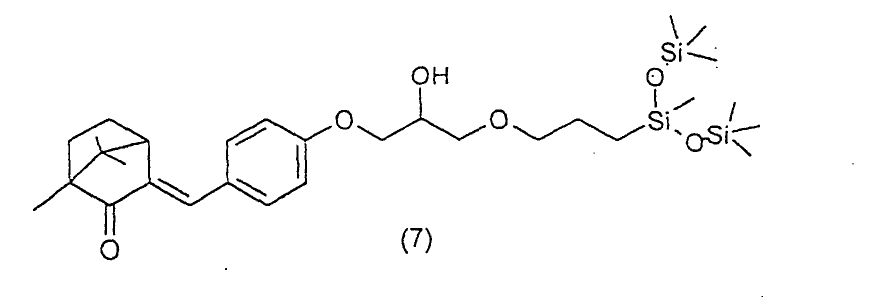

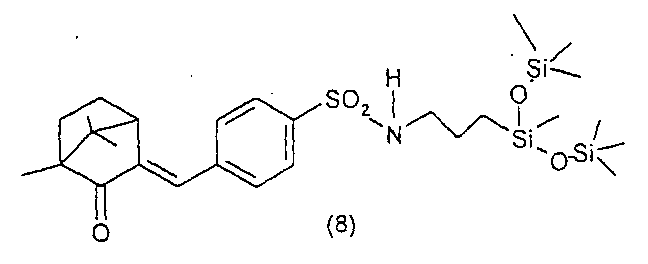

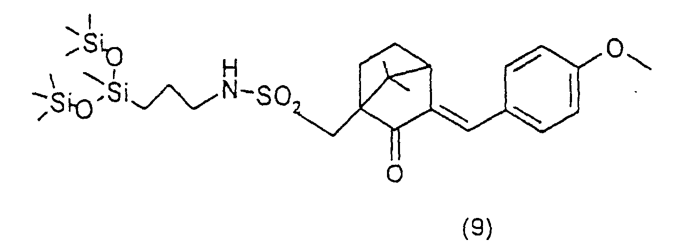

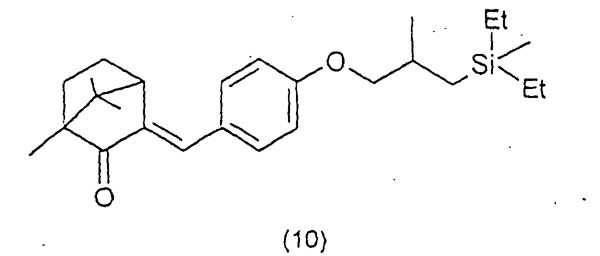

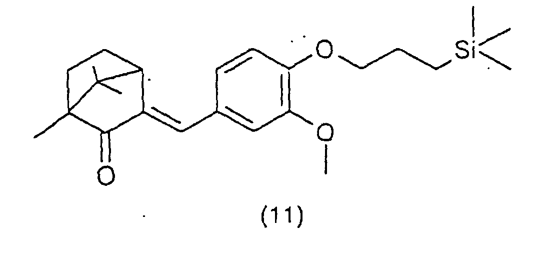

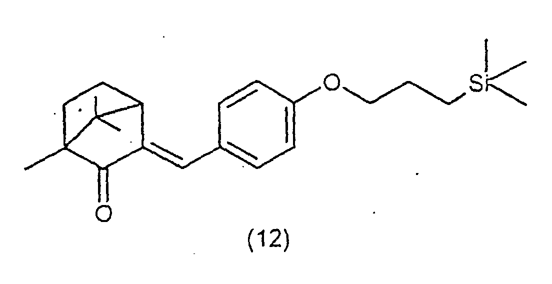

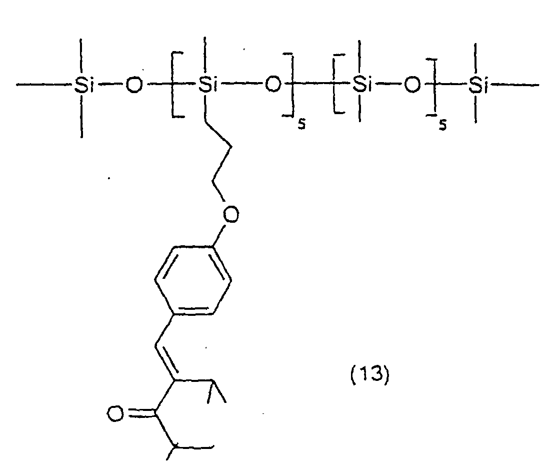

6. Verfahren nach Anspruch 2, dadurch gekennzeichnet, daß die Verbindung der Formel (I) unter den Verbindungen ausgewählt ist, worin p 1 ist und die Gruppe Z eine gesättigt oder ungesättigte, geradkettige oder verzweigte C₁₋₆-Diylgruppe ist, die eine Hydroxygruppe aufweist.

7. Verfahren nach Anspruch 7, wobei die Verbindung der Formel (I) die Verbindung mit der folgenden Struktur ist:

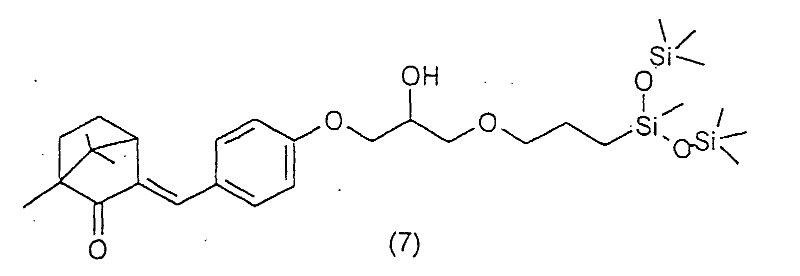

8. Verfahren nach Anspruch 8, wobei die Verbindung der Formel (I) unter den folgenden Verbindungen ausgewählt ist:

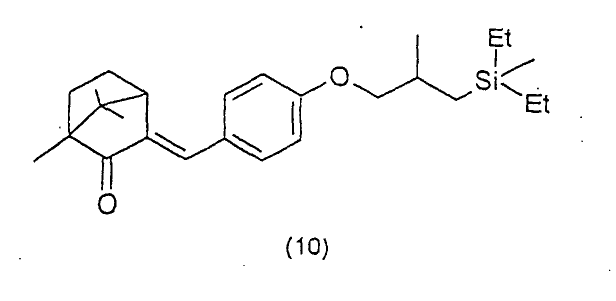

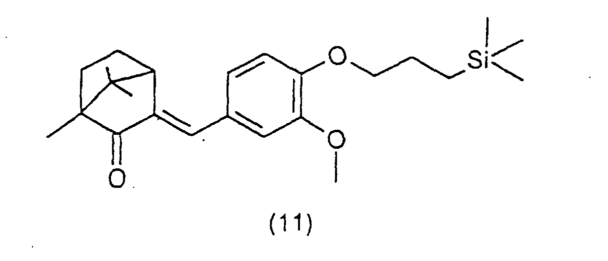

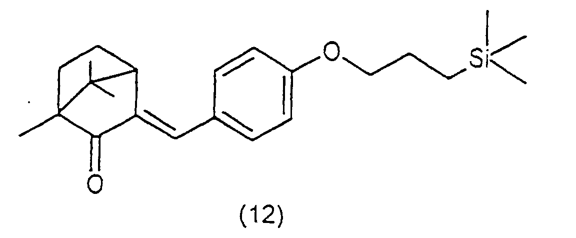

9. Verfahren nach einem der Ansprüche 1 bis 9, dadurch gekennzeichnet, daß das Dibenzoylmethanderivat ausgewählt ist unter:
- 2-Methyl-dibenzoylmethan,
- 4-Methyl-dibenzoylmethen,
- 4-Isopropyl-dibenzoylmethan,
- 4-*tert*.-Butyl-dibenzoylmethan,
- 2,4-Dimethyl-dibenzoylmethan,
- 2,5-Dimethyl-dibenzoylmethan,
- 4,4'-Diisopropyl-dibenzoylmethan,
- 4,4'-Dimethoxy-dibenzoylmethan,
- 4-*tert*.-Butyl-4'-methoxy-dibenzoylmethan,
- 2-Methyl-5-isopropyl-4'-methoxy-dibenzoylmethan,
- 2-Methyl-5-*tert.*-butyl-4'-methoxy-dibenzoylmethan,
- 2,4-Dimethyl-4'-methoxy-dibenzoylmethan, und
- 2,6-Dimethyl-4-*tert*.-butyl-4'-methoxy-dibenzoylmethan.

10. Verfahren nach Anspruch 10, dadurch gekennzeichnet, daß das Dibenzoylmethanderivat das 4-*tert*.-Butyl-4'-methoxy-dibenzoylmethan ist.

11. Verfahren nach Anspruch 10, dadurch gekennzeichnet, daß das Dibenzoylmethanderivat das 4-Isopropyl-dibenzoylmethan ist.

12. Kosmetische, photostabile, filternde Zusammensetzung zum Lichtschutz der Haut und/oder der Haare gegen UV-Strahlung und insbesondere Sonnenlicht auf topischem Wege, die in einem kosmetisch akzeptablen Träger mindestens ein Dibenzoylmethanderivat enthält, dadurch gekennzeichnet, daß sie ferner eine wirksame Menge mindestens einer Silan- oder Organosiloxanverbindung mit Benzylidencamphergruppe enthält.

13. Zusammensetzung nach Anspruch 13, dadurch gekennzeichnet, daß das Dibenzoylmethanderivat ausgewählt ist unter:
- 2-Methyl-dibenzoylmethan,
- 4-Methyl-dibenzoylmethen,
- 4 -Isopropyl-dibenzoylmethan,
- 4-*tert*.-Butyl-dibenzoylmethan,
- 2,4-Dimethyl-dibenzoylmethan,
- 2,5-Dimethyl-dibenzoylmethan,
- 4,4'-Diisopropyl-dibenzoylmethan,
- 4,4'-Dimethoxy-dibenzoylmethan,
- 4-*tert.*-Butyl-4'-methoxy-dibenzoylmethan,
- 2-Methyl-5-isopropyl-4'-methoxy-dibenzoylmethan,
- 2-Methyl-5-*tert.*-butyl-4'-methoxy-dibenzoylmethan,
- 2,4-Dimethyl-4'-methoxy-dibenzoylmethan, und
- 2,6-Dimethyl-4-*tert*.-butyl-4'-methoxy-dibenzoylmethan.

14. Zusammensetzung nach Anspruch 14, dadurch gekennzeichnet, daß das Dibenzoylmethanderivat das 4-*tert*.-Butyl-4'-methoxy-dibenzoylmethan ist.

15. Zusammensetzung nach Anspruch 14, dadurch gekennzeichnet, daß das Dibenzoylmethanderivat das 4-Isopropyl-dibenzoylmethan ist.

16. Zusammensetzung nach einem der Ansprüche 14 bis 17, dadurch gekennzeichnet, daß der Mengenanteil des Dibenzoylmethanderivats oder der Dibenzoylmethanderivate im Bereich von 0,01 bis 10 Gew.-%, bezogen auf das Gesamtgewicht der Zusammensetzung, liegt.

17. Zusammensetzung nach Anspruch 17, dadurch gekennzeichnet, daß der Mengenanteil im Bereich von 0,1 bis 6 Gew.-%, bezogen auf das Gesamtgewicht der Zusammensetzung, liegt.

18. Zusammensetzung nach einem der Ansprüche 13 bis 18, dadurch gekennzeichnet, daß die Silan- oder Organosiloxanverbindung mit Benzylidencamphergruppe der folgenden Formel (I) entspricht: 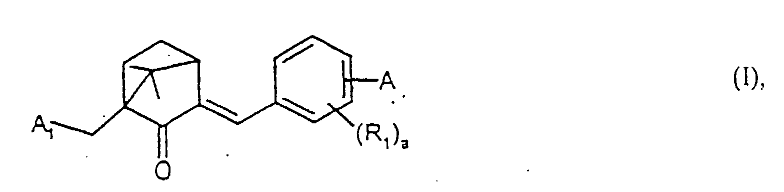 worin bedeuten:
- die Gruppen R₁, die identisch oder voneinander verschieden sind, Wasserstoff, die Gruppe OH, eine gesättigte oder ungesättigte, geradkettige oder verzweigte C₁₋₁₀-Alkylgruppe, eine geradkettige oder verzweigte C₁₋₁₀-Alkoxygruppe oder -OSi(CH₃)₃, wobei zwei angrenzende Gruppen R₁ eine Alkylidendioxygruppe bilden können, worin die Alkylidengruppe 1 bis 2 Kohlenstoffatome aufweist;
- a eine ganze Zahl von 1 bis 3;
- A Wasserstoff oder eine Gruppe -L-W:
- L eine zweiwertige Gruppe der folgenden Formeln (IIa) und (IIb):
-CH₂-CHR₂-[C(R₃)₂]ₘ-(CO)ₙ-(O)ₒ-(Z)ₚ-(Y)_{q}- (IIa)
-CH=CR₂-[C(R₃)₂]ₘ-(CO)ₙ-(O)ₒ-(Z)ₚ-(Y)_{q}- (IIb)
worin bedeuten:
- R₂ und R₃, die identisch oder voneinander verschieden sind, Wasserstoff oder eine geradkettige oder verzweigte C₁₋₅-Alkylgruppe,
- Z eine geradkettige oder verzweigte, gesättigte oder ungesättigte, gegebenenfalls mit einer Hydroxygruppe oder einer geradkettigen oder verzweigten, gesättigten oder ungesättigten C₂₋₈-Alkylgruppe substituierte C₁₋₆-Diylgruppe,
- Y eine Gruppe -O-, -NR₄-, -SO₂NH-, -(CO)O-, -(CO)NH- oder -O(CO)NH-, wobei R₄ Wasserstoff oder eine C₁₋₅-Alkylgruppe bedeutet,
- m Null oder eine ganze Zahl von 1 bis 10,
- n 0 oder 1,
- o 0 oder 1,
- p 0 oder 1,
- q 0 oder 1,
- A₁ Wasserstoff oder eine Gruppe -L₁-W, wobei L₁ der Definition von L entspricht, mit der Maßgabe, daß Y -SO₂NH- bedeutet, wenn q = 1;
- mit der Maßgabe, daß nur eine der beiden Gruppen A und A₁ Wasserstoff bedeutet;
- W eine Gruppe der folgenden Formeln (1), (2) oder (3): 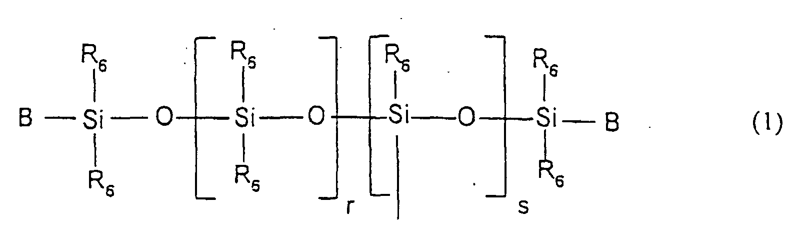 oder 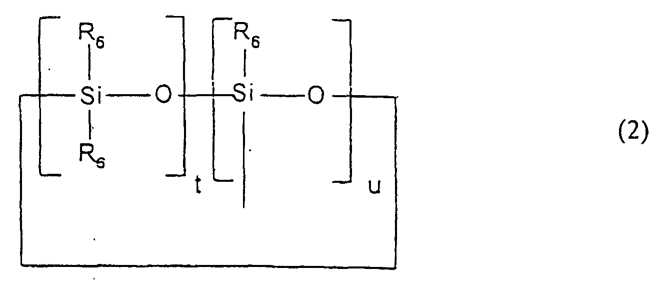 oder
Si(R₆)₃ (3),
worin:
- die Gruppen R₆, die identisch oder voneinander verschieden sind, unter den geradkettigen oder verzweigten C₁₋₃₀-Alkylgruppen und Phenyl ausgewählt sind, wobei mindestens 80 % der Gruppen R₆ Methyl bedeuten,
- die Gruppen B, die identisch oder voneinander verschieden sind, unter den Gruppen R₆ und der Gruppe X der folgenden Formel ausgewählt sind: 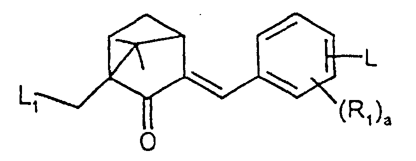 wobei R₁, L, L₁ und a die oben angegebenen Bedeutungen aufweisen;
- r 0 oder eine ganze Zahl im Bereich von 1 bis 200 und s 0 oder eine ganze Zahl im Bereich von 1 bis 50 bedeutet, und mindestens eine der beiden Gruppen B X bedeutet, wenn s = 0,
- u eine ganze Zahl im Bereich von 1 bis 10 und t 0 oder eine ganze Zahl im Bereich von 1 bis 10 ist, mit der Maßgabe, daß t + u 3 bedeutet oder darüber liegt.

19. Zusammensetzung nach Anspruch 19, dadurch gekennzeichnet, daß die Verbindung der Formel (I) mindestens eine der folgenden Eigenschaften aufweist:
- R₁ = H, OCH₃, CH₃ oder zwei angrenzende Gruppen R₁ bilden Methylendioxy,
- R₂ = H oder CH₃,
- n = 0,
- q = 1.

20. Zusammensetzung nach Anspruch 19, dadurch gekennzeichnet, daß die Verbindung der Formel (I) mindestens eine der folgenden Eigenschaften aufweist:
- R₆ = Methyl,
- B = Methyl.
- r liegt im Bereich von 5 bis 20,
- s liegt im Bereich von 2 bis 15,
- t + u liegt im Bereich von 3 bis 10,
- m = 1,
- R₃ = Wasserstoff oder Methyl.

21. Zusammensetzung nach Anspruch 19, dadurch gekennzeichnet, daß die Verbindung der Formel (I) unter den Verbindungen mit s = 0 ausgewählt ist.

22. Zusammensetzung nach Anspruch 19, dadurch gekennzeichnet, daß die Verbindung der Formel (I) unter den Verbindungen der folgenden Formeln ausgewählt ist:

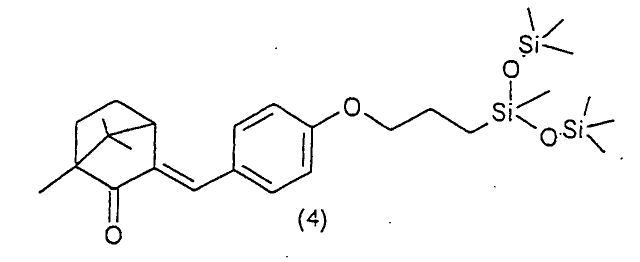

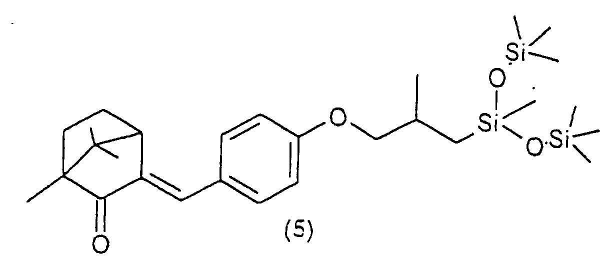

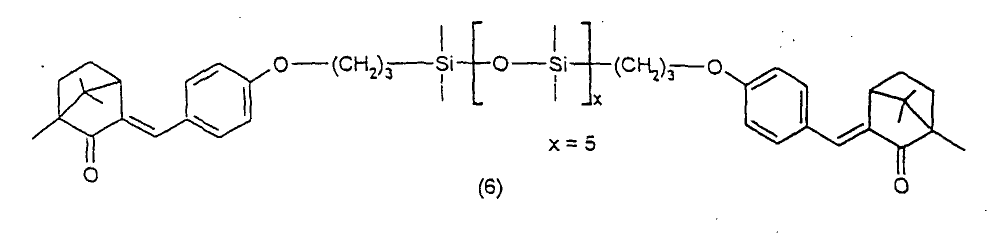

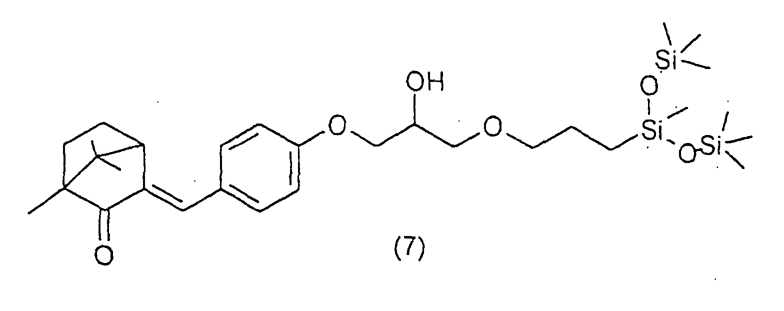

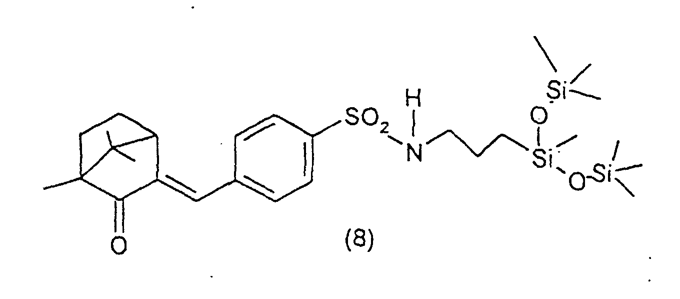

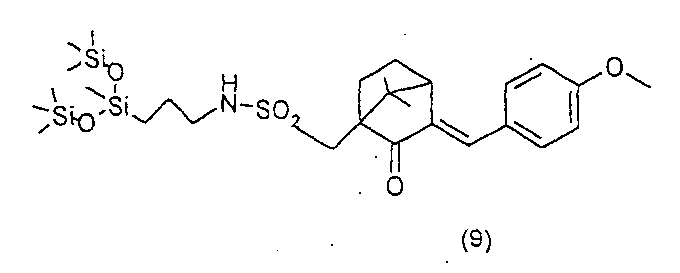

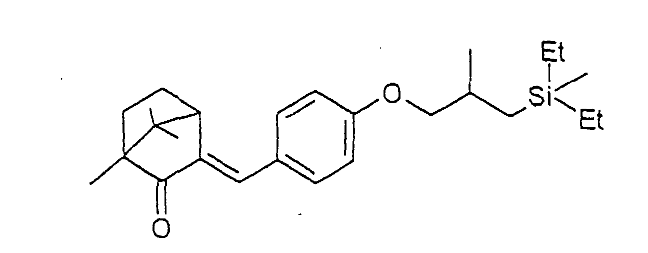

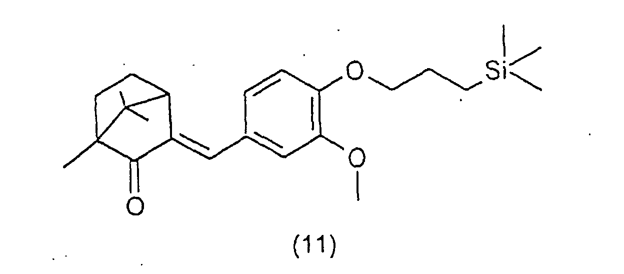

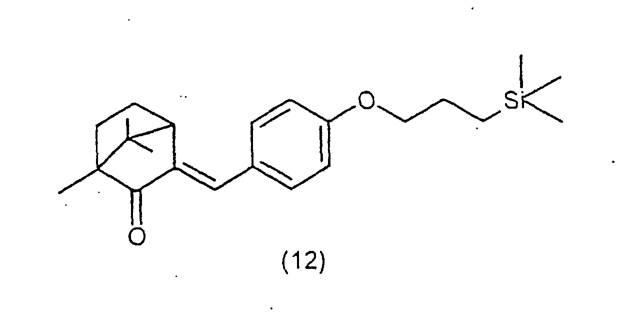

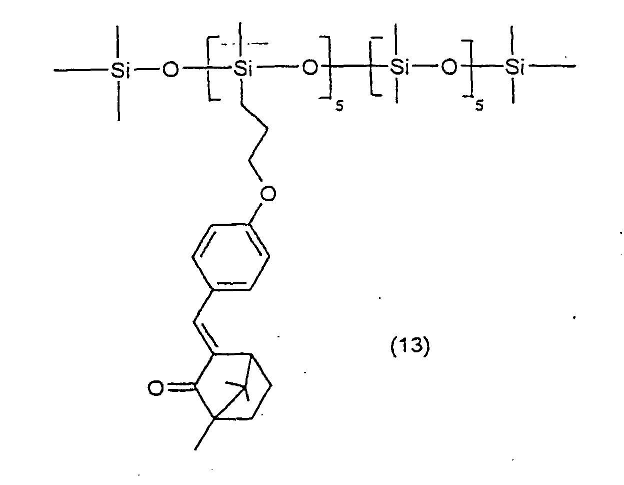

23. Zusammensetzung nach Anspruch 19, dadurch gekennzeichnet, daß die Verbindung der Formel (I) unter den Verbindungen ausgewählt ist, worin p 1 ist und die Gruppe Z eine gesättigte oder ungesättigte, geradkettige oder verzweigte C₁₋₆-Diylgruppe ist, die eine Hydroxygruppe aufweist.

24. Zusammensetzung nach einem der Ansprüche 13 bis 24, dadurch gekennzeichnet, daß der Mengenanteil der Silan- oder Organosiloxanverbindung(en) mit Benzylidencamphergruppe mindestens 0,5 Gew.-%, bezogen auf das Gesamtgewicht der Zusammensetzung, beträgt.

25. Zusammensetzung nach Anspruch 25, dadurch gekennzeichnet, daß der Mengenanteil im Bereich von 0,5 bis 20 Gew.-%, bezogen auf das Gesamtgewicht der Zusammensetzung, liegt.

26. Verbindungen der folgenden Formel (I): 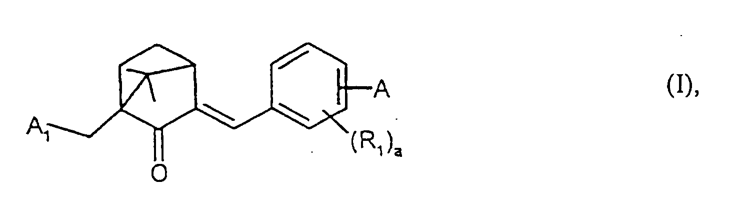 worin bedeuten:
- die Gruppen R₁, die identisch oder voneinander verschieden sind, Wasserstoff, die Gruppe OH, eine gesättigte oder ungesättigte, geradkettige oder verzweigte C₁₋₁₀-Alkylgruppe, eine geradkettige oder verzweigte C₁₋₁₀-Alkoxygruppe oder -OSi(CH₃)₃, wobei zwei angrenzende Gruppen R₁ gemeinsam eine Alkylidendioxygruppe bilden können, worin die Alkylidengruppe 1 bis 2 Kohlenstoffatome aufweist;
- a eine ganze Zahl von 1 bis 3;
- A Wasserstoff oder eine Gruppe -L-W:
- L eine zweiwertige Gruppe der folgenden Formeln (IIa) oder (IIb):
-CH₂-CHR₂-[C(R₃)₂]ₘ-(CO)ₙ-(O)ₒ-(Z)ₚ-(Y)_{q}- (IIa)
-CH=CR₂-[C(R₃)₂]ₘ-(CO)ₙ-(O)ₒ-(Z)ₚ-(Y)_{q}- (IIb)
worin bedeuten:
- R₂ und R₃, die identisch oder voneinander verschieden sind, Wasserstoff oder eine geradkettige oder verzweigte C₁₋₅-Alkylgruppe,
- Z eine geradkettige oder verzweigte, gesättigte oder ungesättigte, gegebenenfalls mit einer Hydroxygruppe oder einer geradkettigen oder verzweigten, gesättigten oder ungesättigten C₂₋₈-Alkylgruppe substituierte C₁₋₆-Diylgruppe,
- Y eine Gruppe -O-, -NR₄-, -SO₂NH-, -(CO)O-, -(CO)NH- oder -O(CO)NH-, wobei R₄ Wasserstoff oder eine C₁₋₅-Alkylgruppe bedeutet,
- m Null oder eine ganze.Zahl von 1 bis 10,
- n 0 oder 1,
- o 0 oder 1,
- p 0 oder 1,
- q 0 oder 1,
- A₁ Wasserstoff oder eine Gruppe -L₁-W, wobei L₁ der Definition von L entspricht, mit der Maßgabe, daß Y -SO₂NH- bedeutet, wenn q = 1;
- mit der Maßgabe, daß nur eine der beiden Gruppen A und A₁ Wasserstoff bedeutet;
- W eine Gruppe der folgenden Formeln (1), (2) oder (3): 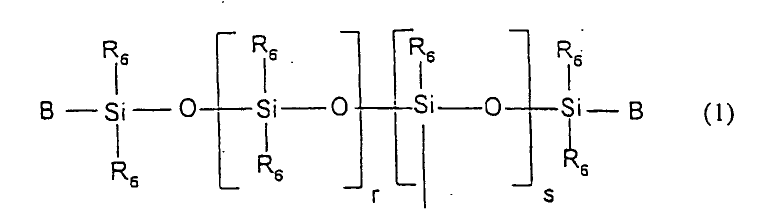 oder 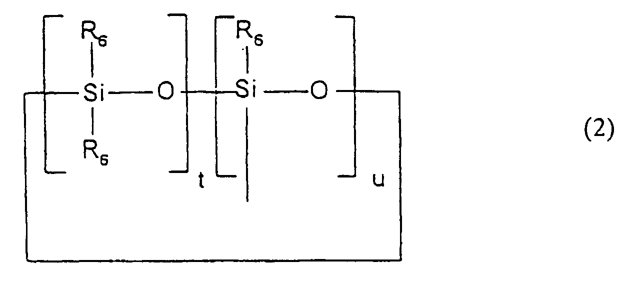 oder
Si(R₆)₃ (3),
worin:
- die Gruppen R₆, die identisch oder voneinander verschieden sind, unter den geradkettigen oder verzweigten C₁₋₃₀-Alkylgruppen und Phenyl ausgewählt sind, wobei mindestens 80 % der Gruppen R₆ Methyl bedeuten,
- die Gruppen B, die identisch oder voneinander verschieden sind, unter den Gruppen R₆ und der Gruppe X der folgenden Formel ausgewählt sind: 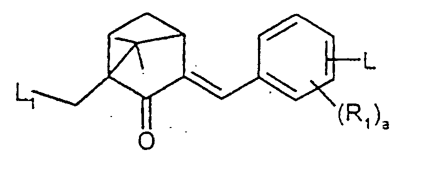 wobei R₁, L, L₁ und a die oben angegebenen Bedeutungen aufweisen;
- r 0 oder eine ganze Zahl im Bereich von 1 bis 200 und s 0 oder eine ganze Zahl im Bereich von 1 bis 50 bedeutet, und mindestens eine der beiden Gruppen B X bedeutet, wenn s = 0,
- u eine ganze Zahl im Bereich von 1 bis 10 und t 0 oder eine ganze Zahl im Bereich von 1 bis 10 ist, mit der Maßgabe, daß t + u 3 bedeutet oder darüber liegt.

27. Verbindung der Struktur:

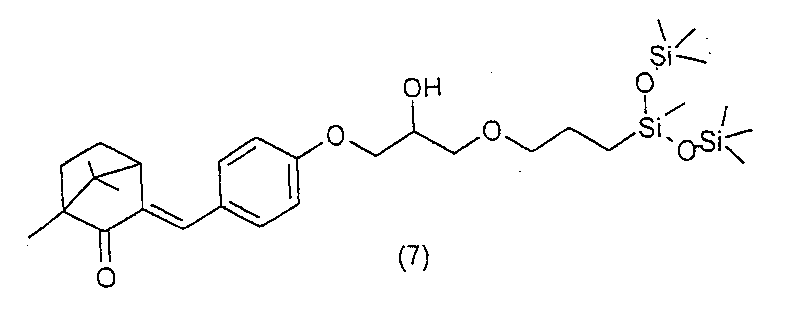

28. Verbindung der Struktur:

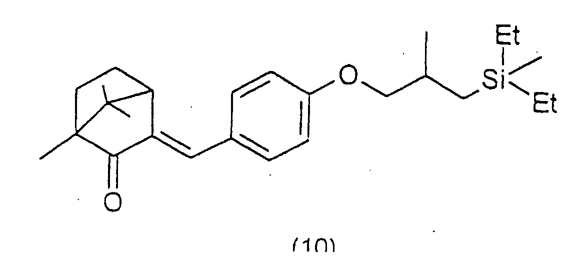

29. Verbindung der Struktur:

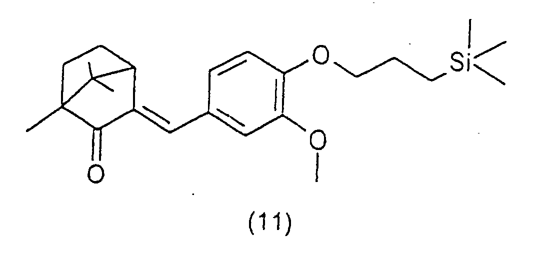

30. Verbindung der Struktur:

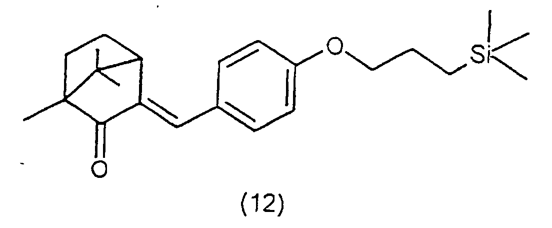

31. Verwendung einer Silan- oder Organosiloxanverbindung mit Benzylidencamphergruppe nach einem der Ansprüche 1 bis 9, 27 bis 31 zur Herstellung einer kosmetischen oder dermatologischen Zusammensetzung, die mindestens ein Dibenzoylmethanderivat enthält, zur Verbesserung der Photostabilität des Dibenzoylmethanderivats gegenüber UV-Strahlung.

## Claims

1. Process for improving the stability of at least one dibenzoylmethane derivative with respect to UV radiation, characterized in that it consists in combining an effective. amount of a silane or organosiloxane compound containing a benzylidenecamphor function with the said dibenzoylmethane derivative.

2. Process according to Claim 1, characterized in that the silane or organosilane compound containing a benzylidenecamphor function corresponds to formula (I) below: 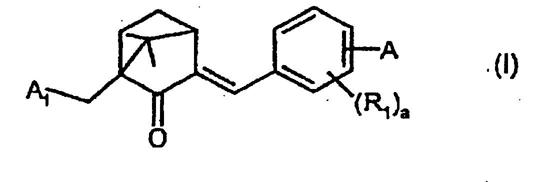 in which:
- R₁, which may be identical or different, represent hydrogen, an OH radical, a saturated or unsaturated, linear or branched, C₁-C₁₀ alkyl radical, a linear or branched C₁-C₁₀ alkoxy radical or an -OSi(CH₃)₃ radical, it being possible for two adjacent groups R₁ together to form an alkylidenedioxy group in which the alkylidene group contains 1 or 2 carbon atoms;
- a is an integer ranging from 1 to 3;
- A is hydrogen or a radical of formula -L-W such that:
- L is a divalent radical of formula (IIa) or (IIb) below:
-CH₂-CHR₂-[C(R₃)₂]ₘ-(CO)ₙ-(O)ₒ-(Z)ₚ-(Y)_{q}- (IIa)
-CH=CR₂-[C(R₃)₂]ₘ-(CO)ₙ-(O)ₒ-(Z)ₚ-(Y)_{q}- (IIb)
in which:
- R₂ and R₃, which may be identical or different, represent hydrogen or a linear or branched C₁-C₅ alkyl radical,
- Z is a linear or branched, saturated or unsaturated C₁-C₆ diyl radical, optionally substituted with a hydroxyl or linear or branched, saturated or unsaturated C₂-C₈ alkyl radical,
- Y represents -O-, -NR₄-, -SO₂NH-, -(CO)O-, -(CO)NH- or -O(CO)NH-, where R₄ represents hydrogen or a C₁-C₅ alkyl radical;
- m is an integer ranging from 0 to 10;
- n is 0 or 1;
- o is 0 or 1;
- p is 0 or 1;
- q is 0 or 1;
- A₁ is hydrogen or the radical -L₁-W in which the radical L₁ has the definition of L with the proviso that when q = 1, then Y represents -SO₂NH-, and it being understood that only one of the two radicals A and A₁ is hydrogen;
- W is a radical of formula (1), (2) or (3) below: 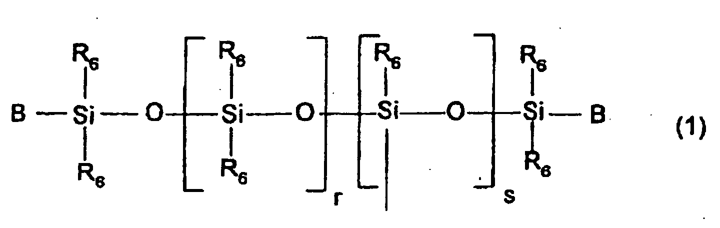 or 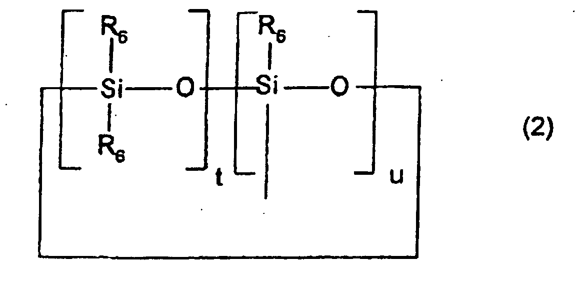
-Si(R₆)₃ (3)
in which:
- R₆, which may be identical or different, are chosen -from linear or branched C₁-C₃₀ alkyl and phenyl radicals, at least 80%, in numerical terms, of the radicals R₆ being methyl;
- B, which may be identical or different, are chosen from the radicals R₆ and the radical X of the formula below: 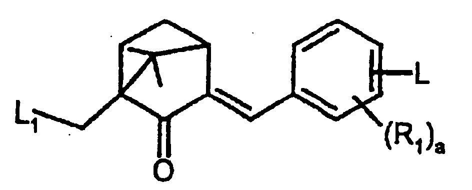 in which R₁, L, L₁ and a have the same meanings as above;
- r is an integer between 0 and 200 inclusive, and s is an integer between 0 and 50 inclusive, and if s = 0, at least one of the two symbols B denotes X;
- u is an integer between 1 and 10 inclusive, and t is an integer between 0 and 10 inclusive, it being understood that t + u is greater than or equal to 3.

3. Process according to Claim 2, characterized in that the compound of formula (I) corresponds to at least one of the following characteristics:
- R₁ = H, OCH₃, CH₃ or two adjacent radicals R₁ form a methylenedioxy,
- R₂ = H or CH₃;
- n = 0;
- q = 1.

4. Process according to Claim 2, characterized in that the compound of formula (I) also corresponds to at least one of the following characteristics:
- R₆ is methyl;
- B is methyl;
- r is between 5 and 20 inclusive;
- s is between 2 and 15 inclusive;
- t + u is between 3 and 10 inclusive;
- m = 1;
- R₃ is hydrogen or a methyl radical.

5. Process according to Claim 2, characterized in that the compound of formula (I) is chosen from those in which s = 0.

6. Process according to Claim 2, characterized in that the compound of formula (I) is chosen from the compounds of the following formulae:

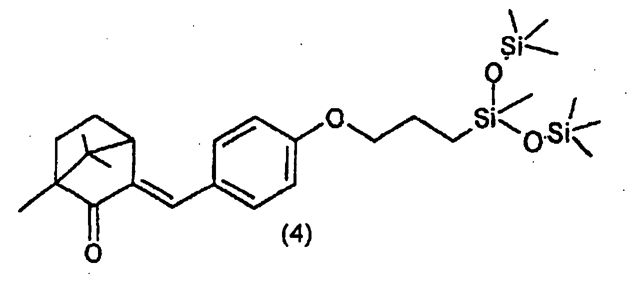

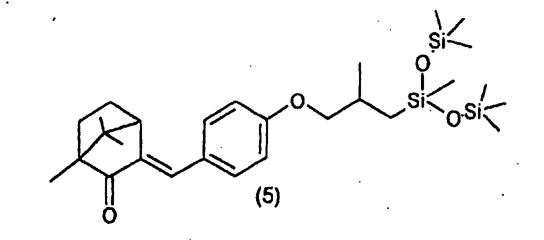

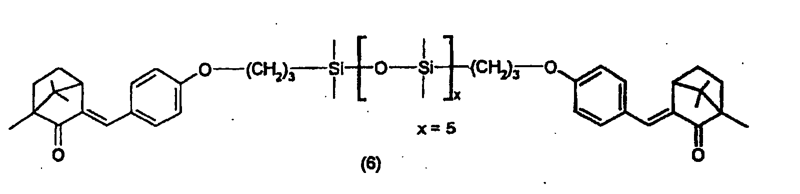

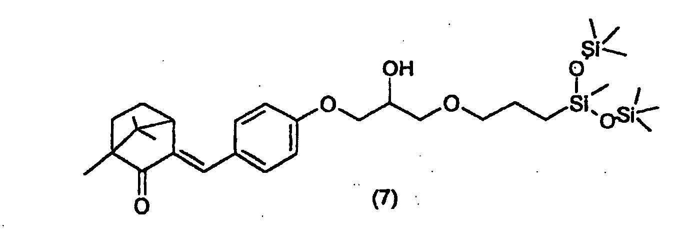

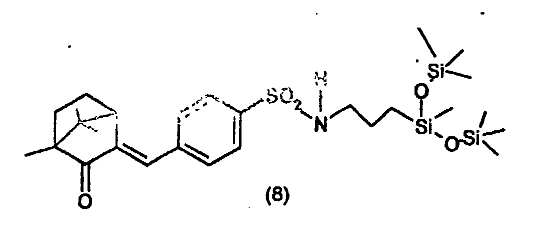

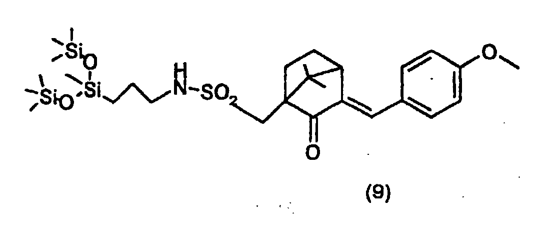

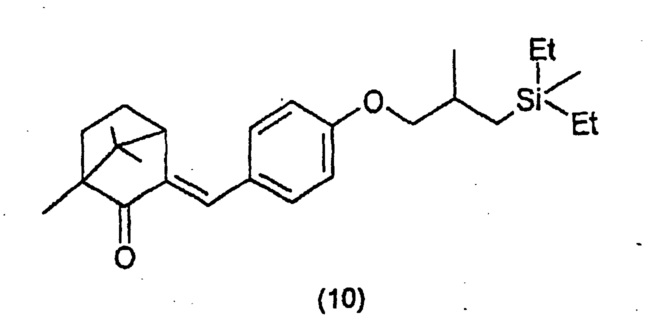

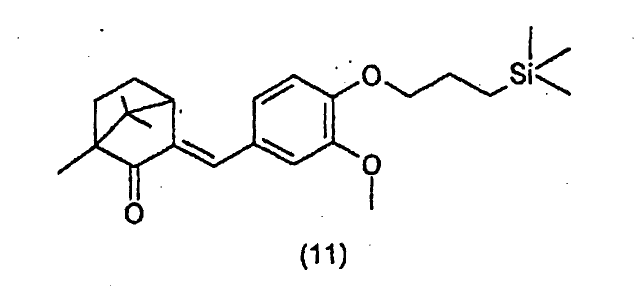

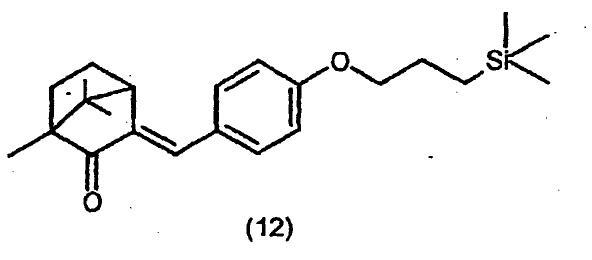

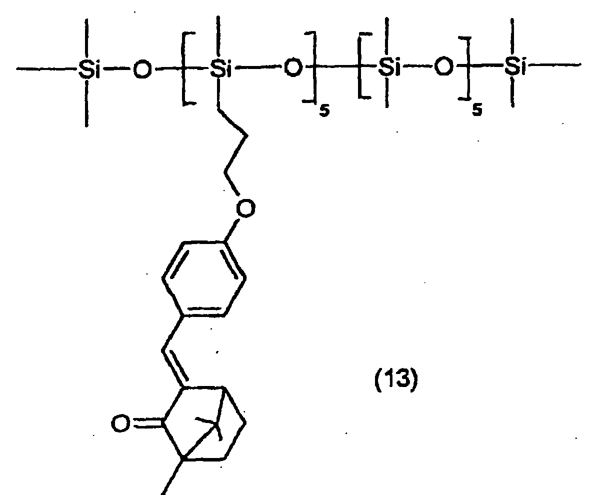

7. Process according to Claim 2, characterized in that the compound of formula (I) is chosen from those in which p is equal to 1 and the radical Z is a linear or branched, saturated or unsaturated C₁-C₆ diyl radical containing a hydroxyl radical.

8. Process according to Claim 7, in which the compound of formula (I) is the compound of structure:

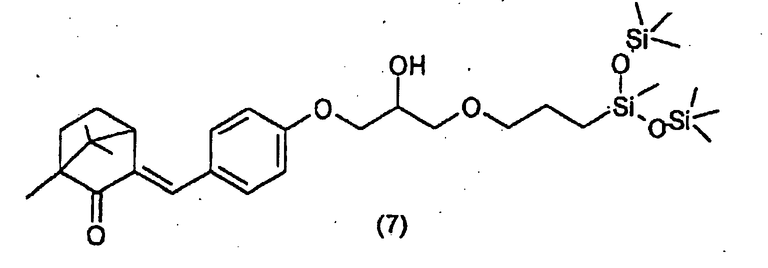

9. Process according to Claim 8, in which the compound of formula (I) is chosen from the group consisting of the following compounds:

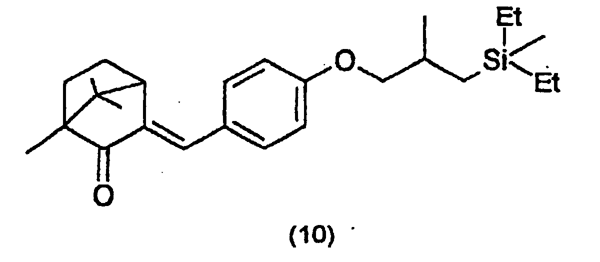

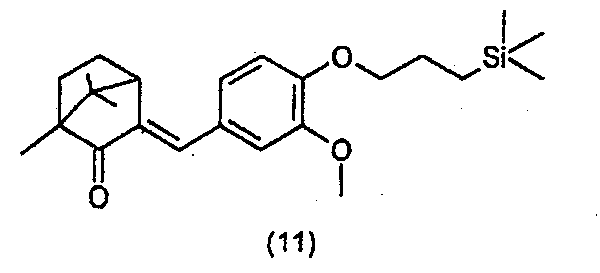

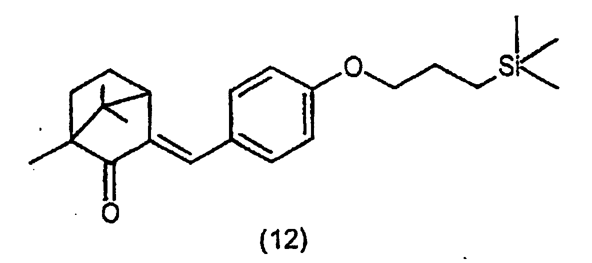

10. Process according to any one of Claims 1 to 9, characterized in that the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2, 5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2, 4-dimethyl-4'-methoxydibenzoylmethane,
- 2, 6-dimethyl-4-tert-butyl-4'-methoxydibenzoylmethane.

11. Process according to Claim 10, characterized in that the dibenzoylmethane derivative is 4-(tert-butyl)-4'-methoxydibenzoylmethane.

12. Process according to Claim 10, characterized in that the dibenzoylmethane derivative is 4-isopropyldibenzoylmethane.

13. Photostable screening cosmetic composition for the topical photoprotection of the skin and/or the hair against ultraviolet radiation, in particular solar radiation, of the type comprising, in a cosmetically acceptable support, at least one dibenzoylmethane derivative, characterized in that it also comprises an effective amount of at least one silane or organosiloxane compound containing a benzylidenecamphor function.

14. Composition according to Claim 13, characterized in that the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2, 5-dimethyldibenzoylmethane,
- 4,4'-diisopropyldibenzoylmethane,
- 4,4'-dimethoxydibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

15. Composition according to Claim 14, characterized in that the dibenzoylmethane derivative is 4- (tert-butyl)-4'-methoxydibenzoylmethane.

16. Composition according to Claim 14, characterized in that the dibenzoylmethane derivative is 4-isopropyldibenzoylmethane.

17. Composition according to any one of Claims 14 to 17, characterized in that the content of dibenzoylmethane derivative(s) is between 0.01% and 10% by weight relative to the total weight of the composition.

18. Composition according to Claim 17, characterized in that the said content is between 0.1% and 6%. by weight relative to the total weight of the composition.

19. Composition according to any one of Claims 13 to 18, in which the silane or organosiloxane compound containing a benzylidenecamphor function corresponds to formula (I) below: 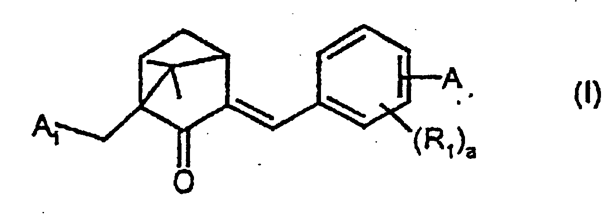 in which:
- R₁, which may be identical or different, represent hydrogen, an OH radical, a saturated or unsaturated, linear or branched, C₁-C₁₀ alkyl radical, a linear or branched C₁-C₁₀ alkoxy radical or an -OSi(CH₃)₃ radical, it being possible for two adjacent groups R₁ together to form an alkylidenedioxy group in which the alkylidene group contains 1 or 2 carbon atoms;
- a is an integer ranging from 1 to 3;
- A is hydrogen or a radical of formula -L-W such that:
- L is a divalent radical of formula (IIa) or (IIb) below:
-CH₂-CHR₂-[C(R₃)₂]ₘ-(CO)ₙ-(O)ₒ-(Z)ₚ-(Y)_{q}- (IIa)
-CH=CR₂-[C(R₃)₂]ₘ-(CO)ₙ-(O)ₒ-(Z)ₚ-(Y)_{q}- (IIb)
in which:
- R₂ and R₃, which may be identical or different, represent hydrogen or a linear or branched C₁-C₅ alkyl radical,
- Z is a linear or branched, saturated or unsaturated C₁-C₆ diyl radical, optionally substituted with a hydroxyl or linear or branched, saturated or unsaturated C₂-C₈ alkyl radical,
- Y represents -O-, -NR₄-, -SO₂NH-, -(CO)O-, -(CO)NH- or -O(CO)NH-, where R₄ represents hydrogen or a C₁-C₅ alkyl radical;
- m is an integer ranging from 0 to 10;
- n is 0 or 1;
- o is 0 or 1;
- p is 0 or 1;
- q is 0 or 1;
- A₁ is hydrogen or a radical -L₁-W in which the radical L₁ has the definition of L with the proviso that when q = 1, then Y represents -SO₂NH-, and it being understood that only one of the two radicals A and A₁ is hydrogen;
- W is a radical of formula (1), (2) or (3) below: 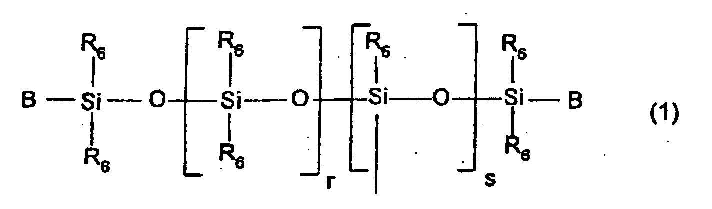 or 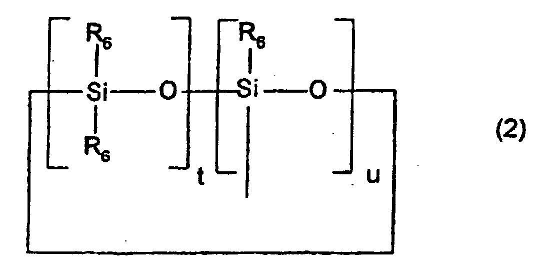
-Si(R₆)₃ (3)
in which:
- R₆, which may be identical or different, are chosen from linear or branched C₁-C₃₀ alkyl and phenyl radicals, at least 80%, in numerical terms, of the radicals R₆ being methyl;
- B, which may be identical or different, are chosen from the radicals R6 and the radical X of the formula below: 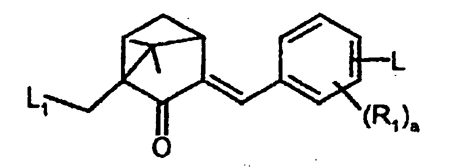 in which R₁, L, L₁ and a have the same meanings as above;
- r is an integer between 0 and 200 inclusive, and s is an integer between 0 and 50 inclusive, and if s = 0, at least one of the two symbols B denotes X;
- u is an integer between 1 and 10 inclusive, and t is an integer between 0 and 10 inclusive, it being understood that t + u is greater than or equal to 3.

20. Composition according to Claim 19, characterized in that the compound of formula (I) corresponds to at least one of the following characteristics:
- R₁ = H, OCH₃, CH₃ or two adjacent radicals R₁ form a methylenedioxy,
- R₂ = H or CH₃;
- n = 0;
- q = 1.

21. Composition according to Claim 19, characterized in that the compound of formula (I) also corresponds to at least one of the following characteristics:
- R₆ is methyl;
- B is methyl;
- r is between 5 and 20 inclusive;
- s is between 2 and 15 inclusive;
- t + u is between 3 and 10 inclusive;
- m = 1;
- R₃ is hydrogen or a methyl radical.

22. Composition according to Claim 19, characterized in that the compound of formula (I) is chosen from those in which s = 0.

23. Composition according to Claim 19, characterized in that the compound of formula (I) is chosen from the compounds of the following formulae:

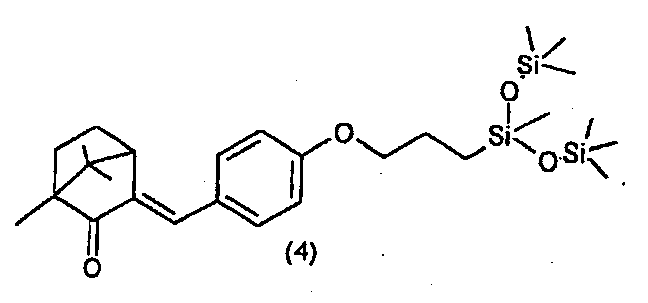

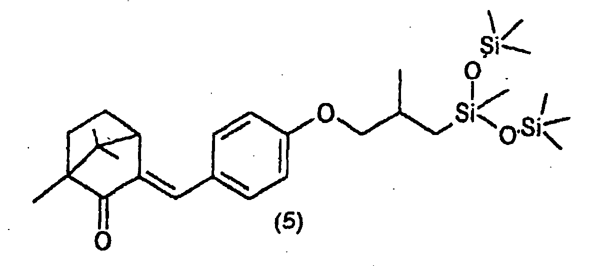

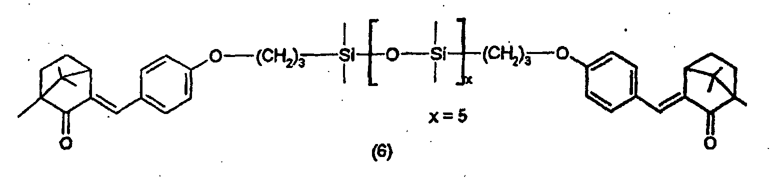

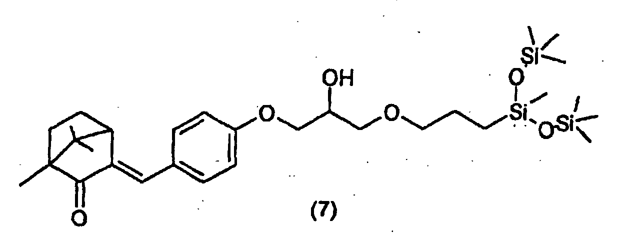

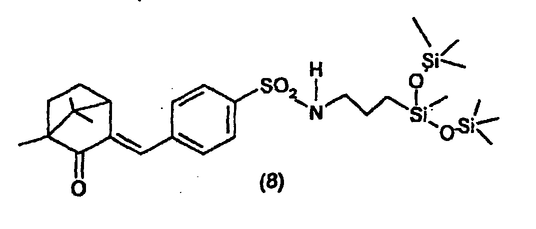

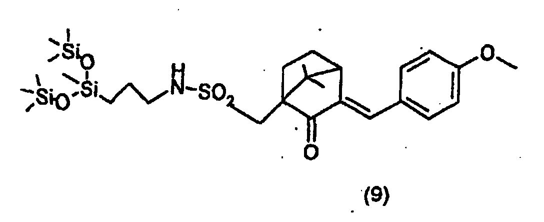

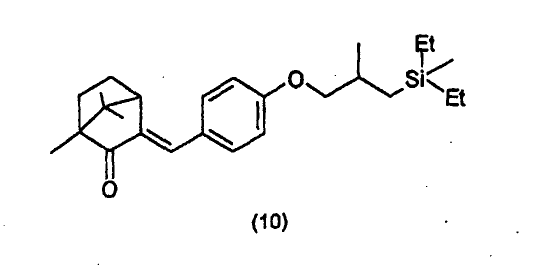

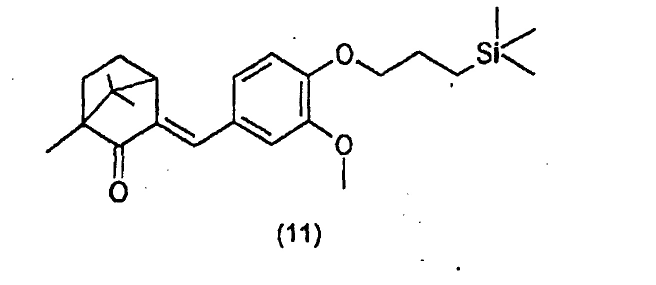

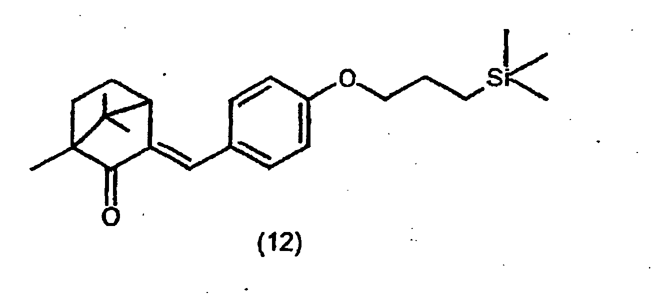

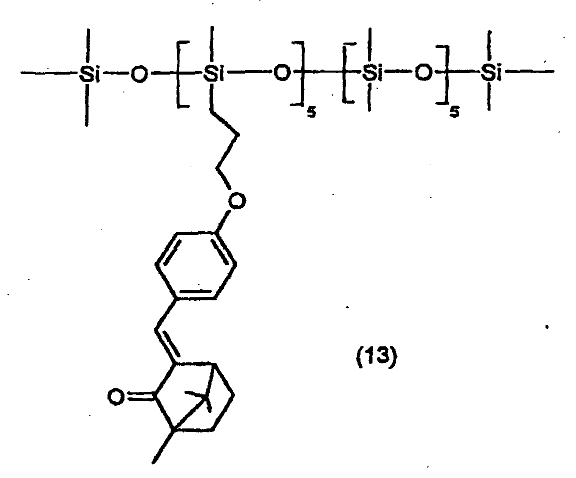

24. Composition according to Claim 19, characterized in that the compound of formula (I) is chosen from those in which p is equal to 1 and the radical Z is a linear or branched, saturated or unsaturated C₁-C₆ diyl radical containing a hydroxyl radical.

25. Composition according to any one of Claims 13 to 24, characterized in that the content of silane or organosiloxane compound(s) is at least equal to 0.5% by weight relative to the total weight of the composition.

26. Composition according to Claim 25, characterized in that the said content ranges from 0.5% to 20% by weight relative to the total weight of the composition.

27. Compound corresponding to formula (I) below: 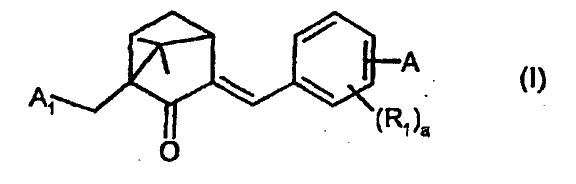 in which:
- R₁, which may be identical or different, represent hydrogen, an OH radical, a saturated or unsaturated, linear or branched, C₁-C₁₀ alkyl radical, a linear or branched C₁-C₁₀ alkoxy radical or an -OSi(CH₃)₃ radical, it being possible for two adjacent groups R₁ together to form an alkylidenedioxy group in which the alkylidene group contains 1 or 2 carbon atoms;
- a is an integer ranging from 1 to 3;
- A is hydrogen or a radical of formula -L-W such that:
- L is a divalent radical of formula (IIa) or (IIb) below:
-CH₂-CHR₂-[C(R₃)₂]ₘ-(CO)ₙ-(O)ₒ-Z-(Y)_{q}- (IIa)
-CH=CR₂-[C(R₃)₂]ₘ-(CO)ₙ-(O)ₒ-Z-(Y)_{q}- (IIb)
in which:
- R₂ and R₃, which may be identical or different, represent hydrogen or a linear or branched C₁-C₅ alkyl radical,
- Z is a linear or branched, saturated or unsaturated C₁-C₆ diyl radical, containing a hydroxyl radical,
- Y represents -O-, -NR₄-, -SO₂NH-, -(CO)O-, -(CO)NH- or -O(CO)NH-, where R₄ represents hydrogen or a C₁-C₅ alkyl radical;
- m is an integer ranging from 0 to 10;
- n is 0 or 1;
- o is 0 or 1;
- q is 0 or 1;
- A₁ is hydrogen or a radical -L₁-W in which the radical L₁ has the definition of L with the proviso that when q .= 1, then Y represents -SO₂NH-, and it being understood that only one of the two radicals A and A₁ is hydrogen;
- W is a radical of formula (1), (2) or (3) below: 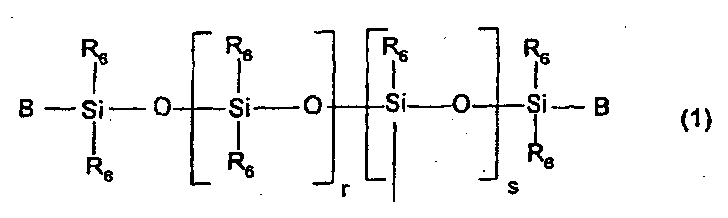 or 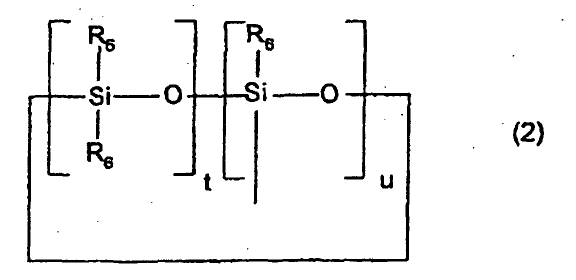
-Si(R₆)₃ (3)
in which:
- R₆, which may be identical or different, are chosen from linear or branched C₁-C₃₀ alkyl and phenyl radicals, at least 80%, in numerical terms,. of the radicals R₆ being methyl;
- B, which may be identical or different, are chosen from the radicals R₆ and the radical X of the formula below: 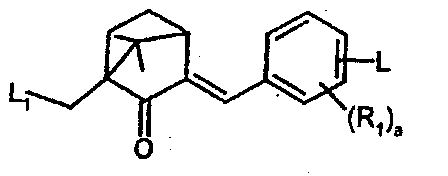 in which R₁, L, L₁ and a have the same meanings as above;
- r is an integer between 0 and 200 inclusive, and s is an integer between 0 and 50 inclusive, and if s = 0, at least one of the two symbols B denotes X;
- u is an integer between 1 and 10 inclusive, and t is an integer between 0 and 10 inclusive, it being understood that t + u is greater than or equal to 3.

28. Compound of structure:

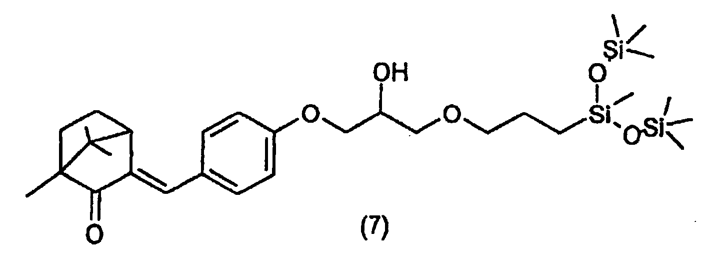

29. Compound of structure

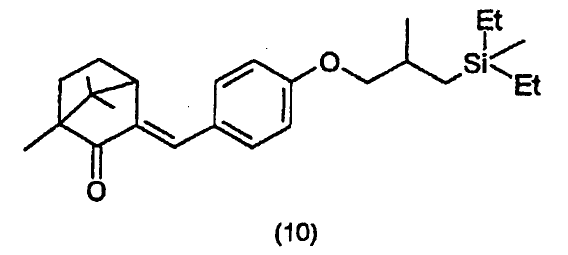

30. Compound of structure:

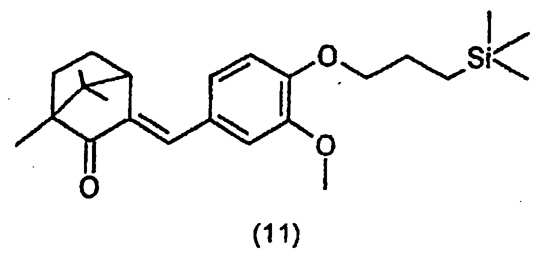

31. Compound of structure:

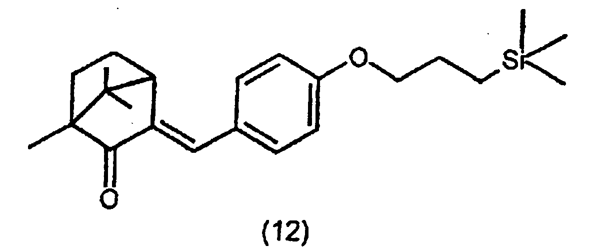

32. Use of a silane or organosiloxane compound containing a benzylidenecamphor function as defined in any one of Claims 1 to 9 and 27 to 31, in the preparation of a cosmetic or dermatological composition containing at least one dibenzoylmethane derivative, in order to improve the stability of the said dibenzoylmethane derivative with respect to UV radiation.
